# EUROPEAN PATENT APPLICATION

(11) **EP 0 585 500 A1**
(43) Date of publication of application: **09.03.1994**
(21) Application number: 92402435.9
(22) Date of filing: 04.09.1992
(51) Int. Cl.: C07D 295/14, C07D 213/75, C07D 233/61, C07D 521/00, C07D 233/24, A61K 31/495

(54) **Diaryl piperazineacetamides as antimuscarinic agents**

(71) Applicant: MERRELL DOW PHARMACEUTICALS INC., Cincinnati Ohio 45215-6300 (US)
(72) Inventor: van Hijfte, Luc, F-67520 Marlenheim (FR); Richards, Mary, F-67370 Pfulgriesheim (FR); Hibert, Marcel, F-67114 Eschau (FR); Hoflack, Jan, F-67190 Mutzig (FR); Trumpp-Kallmeyer, Susanne, F-67000 Strasbourg (FR); Marciniak, Gilbert, F-67100 Strasbourg (FR)
(74) Representative: Gillard, Marie-Louise

(57) **Abstract**

Diaryl piperzineacetamide compounds useful as antimuscarinic agents for treating a variety of indications such as Parkinson's disease, motion sickness and for the inhibition of gastric acid secretion.

## Description

The present invention is directed to novel diaryl piperazinoacetamide compounds useful as antimuscarinic agents. Typically, these compounds can be used in the treatment of Parkinson's disease, motion sickness and for the inhibition of gastric acid secretion.

### BACKGROUND OF THE PRESENT INVENTION

Muscarinic receptors are one of two general types of cholinergic receptors and are distributed in the central nervous system and the peripheral organs. Inhibition of the effects of muscarinic agents at these receptors can produce the interruption of parasympathetic and sympathetic cholinergic nerve stimulation, e.g., decreased gastrointestinal motility, decreased gastric secretion, dry mouth, drying of the mucous membranes in general, mydriasis, loss of accommodation, urinary retention, decreased sweating and compensatory cutaneous flush, bronchial and biliary dilation, tachycardia and several central nervous system effects. Since some of these effects are desirable under certain circumstances, antimuscarinic agents, also known as muscarinic antagonists, have been developed in order to treat a wide variety of clinical conditions in the gastrointestinal tract, respiratory tract, cardiovascular system, central nervous system, genito-urinary tract, in ophthalmology and in conjunction with anesthesia. Goodman and Gilman's The Pharmacological Basis of Therapeutics, 8th ed. Pergamon Press 1990, incorporated herein by reference.

For example, antimuscarinics have been used in the treatment of the following conditions: to dilate the pupil in order to facilitate visualization of the optic fundus and to paralyze accommodation; to break adhesion between the iris and lens; to treat acute iritis, uveitis, irdiocylitis and keratitis; to treat malignant glaucoma; preanesthesia medication to decrease excessive secretions;to treat sialorrhea, acute coryza, hay fever and rhinitis; to treat bronchial asthma; suppress vagally mediated bradyarrhythmias, heart bock or cardiac syncope; to relieve urinary frequency and urgency; to control enuresis; to relieve ureteral colic; to treat functional gastrointestinal disorders; to suppress gastric secretions; and to treat Parkinson's disease.

Until the discovery of pirenzepine and subsequent molecular cloning, it was not known that muscarinic receptors consisted of five subtypes designated as M₁ through M₅. All five subtypes of muscarinic receptors are found in the central nervous system. The other tissues in which muscarinic receptors have been found are the heart, smooth muscle, secretory glands and autonomic ganglia.

Pirenzepine, a selective M₁ antimuscarinic compound, was shown to selectively inhibit gastric acid secretion at concentrations which did not affect several other responses to muscarinic agonists. Therefore, by improving the selectivity of antimuscarinic compounds to muscarinic subtype receptors it may be possible to diminish undesirable responses and/or enhance the selectivity of the desired response.

It is an object of the present invention to provide novel compounds useful as therapeutic antimuscarinic agents. Additionally, the present invention provides antimuscarinic compounds which exhibit activity, and in some cases selectivity, for the M₁ receptor. Therapeutically these compounds may be used for a wide variety of conditions and in particular for the treatment of Parkinson's disease, motion sickness, and the inhibition of gastric acid secretion.

### SUMMARY OF THE PRESENT INVENTION

A compound of formula : 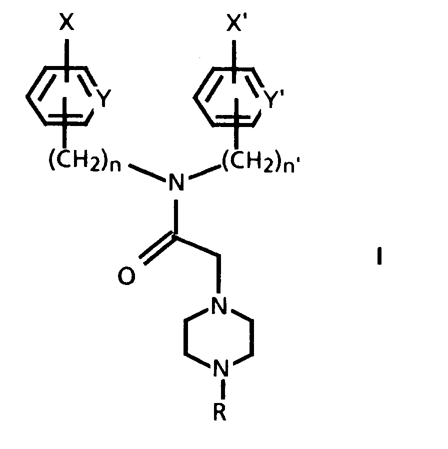
and the pharmaceutically acceptable salts thereof, wherein
each of X and X' are independently -H,-C₁-C₄ alkyl, -Cl, -Br, -I, -F, -(CH₂)ₘCN, -(CH₂)ₘCOOR₁, -(CH₂)ₘCONR₂R₃, CF₃, -(CH₂)ₘNHQ, -(CH₂)ₘO(CH₂)_{p'}Z, -(CH₂)ₘNR₂R₃, -SO₃H, SO₂NR₂R₃, 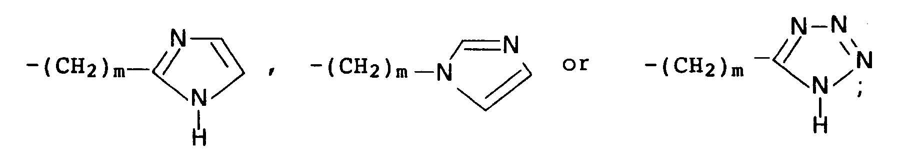
each of Y and Y' are independently -C- or -N-;
each of n, n' or m are independently 0 or 1;
p is 1, 2, 3, or 4;
p' is 0, 1, 2, 3, or 4;
R and R₁ are each independently H or C₁-C₄ alkyl;
each of R₂ and R₃ are independently H, -CH₃, -CH₂CH₃, C₄-C₇ cycloalkyl or taken together with the nitrogen atom form a heterocyclic ring having from 4 to 6 carbon atoms;
R₄ is C₁-C₄ alkyl;
Z is H, OH, -NR₂R₃, -C(O)OR₁, 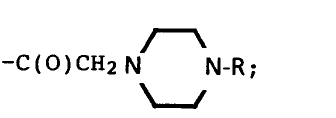
or 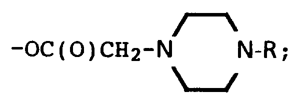
and
Q is -C(O)R₄, -(CH₂)_{p'}C(O)OR₁, -C(O)(CH₂)ₚC(O)OR₁, -C(O)CH=CHC(O)OR₁ or 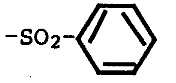
in which the phenyl moiety is optionally substituted with C₁-C₄ alkyl, C₁-C₄ alkoxy, CF₃ or NO₂,
provided that when Y or Y' are -N-, Y and Y' are unsubstituted,
provided that when Y is -N-, X is an ortho -NH₂ or H,
provided that when Y' is -N-, X' is an ortho -NH₂ or H,
provided that when Z is OH, -NR₂R₃ or 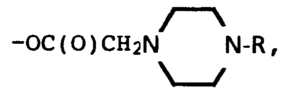
p' is 2, 3, or 4.

The compounds of the present invention may be used to treat conditions responsive to antagonism of muscarinic receptors, and, more preferably, muscarinic receptors of the M₁ receptor subtype. Some conditions responsive to treatment with the compounds of Formula I are motion sickness, Parkinson's disease and conditions responsive to inhibition of gastric acid secretion such as peptic ulcer disease.

### PREFERRED EMBODIMENTS OF THE PRESENT INVENTION

Formula I of the present invention is a N- disubstituted piperazino moiety. One nitrogen of the piperazino moiety is substituted (designated as R) with either hydrogen or a C₁₋₄ alkyl. Preferably, R is C₁₋₄ alkyl, and, more preferably, methyl. The other nitrogen of the piperazino moiety is substituted with an acetamido moiety on which the nitrogen is disubstituted; the disubstitution on the nitrogen does not have to be identical.

Referring to the nitrogen substitution on the acetamido moiety, n or n' can each be zero or one indicating whether or not there is a methylene moiety attached to the nitrogen atom. Preferably, n and n' are the same, and more preferably, n and n' are each zero.

The diaryl structures attached either directly to the nitrogen atom of the acetamido moiety (when n or n' are zero) or attached via a methylene moiety to the nitrogen atom of the acetamido moiety (when n or n' are one) can each be phenylene (Y or Y' are C) or a pyridine moiety (Y or Y' are N). When Y or Y' are N, then the N remains unsubstitued, i.e., when there is a pyridine moiety, the attachment to the remainder of the molecule is not via the nitrogen atom of the pyridine moiety nor is the nitrogen atom substituted with X or X'. Additionally, when Y or Y' are N, respectively X or X' are substituents ortho to N and are limited to hydrogen or amino substituents. It is intended that this ortho substitution does not include substitution at the point of attachment of the ring structures to the rest of the molecule. Y or Y' can be the same or different. Preferably, Y and Y' are the same, and more preferably, Y and Y' are each C.

X or X' can be the same or different and represent substitutents at the same or different positions on the ring structures with the foregoing caveat in the case where Y and/or Y' are N (a nitrogen atom). Preferably, X and X' are different.

It is intended that the generic structure of Formula I represents that X and/or X' are not at the same position on the ring structures as that position which is attached to the rest of the molecule. If the point of attachment of the ring structures to the remainder of the molecule is 1, then each of X and/or X' independently can be 2, 3, 4, 5 or 6. Preferably, X and/or X' are at position number 4, commonly known as the para position.
Each of X and X' are independently -H,-C₁-C₄ alkyl, -Cl, -Br, -I, -F, -(CH₂)ₘCN, -(CH₂)ₘCOOR₁, -(CH₂)ₘCONR₂R₃, CF₃, -(CH₂)ₘNHQ, -(CH₂)ₘO(CH₂)_{p'}Z, -(CH₂)ₘNR₂R₃, -SO₃H, SO₂NR₂R₃, 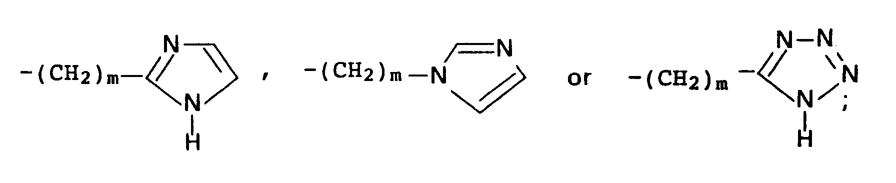
wherein p is 1, 2, 3 or 4; p' is 0,1,2,3 or 4; and m is 0 or 1.
Each of R₂ and R₃ are independently H , -CH₃, -CH₂CH₃, C₄-C₇ cycloalkyl or taken together form a heterocyclic ring having from 4 to 6 carbon atoms;
R₄ is C₁-C₄ alkyl; Z is H, OH, -NR₂R₃, 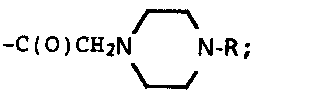
-C(O)OR₁, or 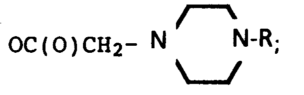
and
Q is -C(O)R₄, -(CH₂)_{p'} C(O)OR₁, -C(O)(CH₂)ₚC(O)OR₁, -C(O)CH=CHC(O)OR₁ or -SO₂-phenyl in which the phenyl moiety is optionally substituted with C₁-C₄ alkyl or alkoxy, CF₃ or NO₂.

Preferably, at least one of X or X' is -(CH₂)ₘNR₂R₃, hydrogen, -(CH₂)ₘNHQ, -(CH₂)ₘO(CH₂)_{p'}Z, or C₁₋₄ alkyl. More preferably, only one of X or X' is H. Preferably, m is 0. Each of R₂ and R₃ are preferably hydrogen, methyl, ethyl or together form a heterocyclic ring having 4 or 6 Carbon atoms. Preferably, R₄ is methyl, and/or R₁ is hydrogen, methyl or tert-butyl. Preferably m is 0 and/or R₂ and R₃ are each H. When Z is other that H or -C(O)OR₁ or 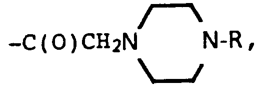
then p' is 2, 3 or 4.

Certain terms as used herein have the defined following meanings:
(1) "C₁₋₄ alkyl" means a straight or branched alkyl moiety having 1, 2, 3 or 4 carbon atoms such as methyl, ethyl, n-propyl, isopropyl, n-butyl and tert-butyl;
(2) "treatment" means to prevent contracting the specified disease or to relieve in part or in whole a patient's disease or the symptoms related therewith;
(3) "patient" means warm-blooded animals, such as for example rats, mice, dogs, cats, guinea pigs, and primates such as humans; and
(4) "optionally substituted" means that the named substituent may or may not be present in the compound of interest, and, if regarding optional substitution of a phenyl ring, if not otherwise designated, the substitution may be mono-, di-, or tri-substitution at any appropriate position on the ring, but preferably at a position other than the position which provides attachment to the remainder of the molecule.

As used herein, the term "Hal", "halo" or "halide" refers to a chlorine, bromine or iodine atom.

The compounds of formula (I) may be prepared using techniques and procedures well known and appreciated by one of ordinary skill in the art. A general synthetic procedure for preparing these compounds is set forth in Scheme A. In Scheme A, all substituents unless otherwise indicated are as previously defined. 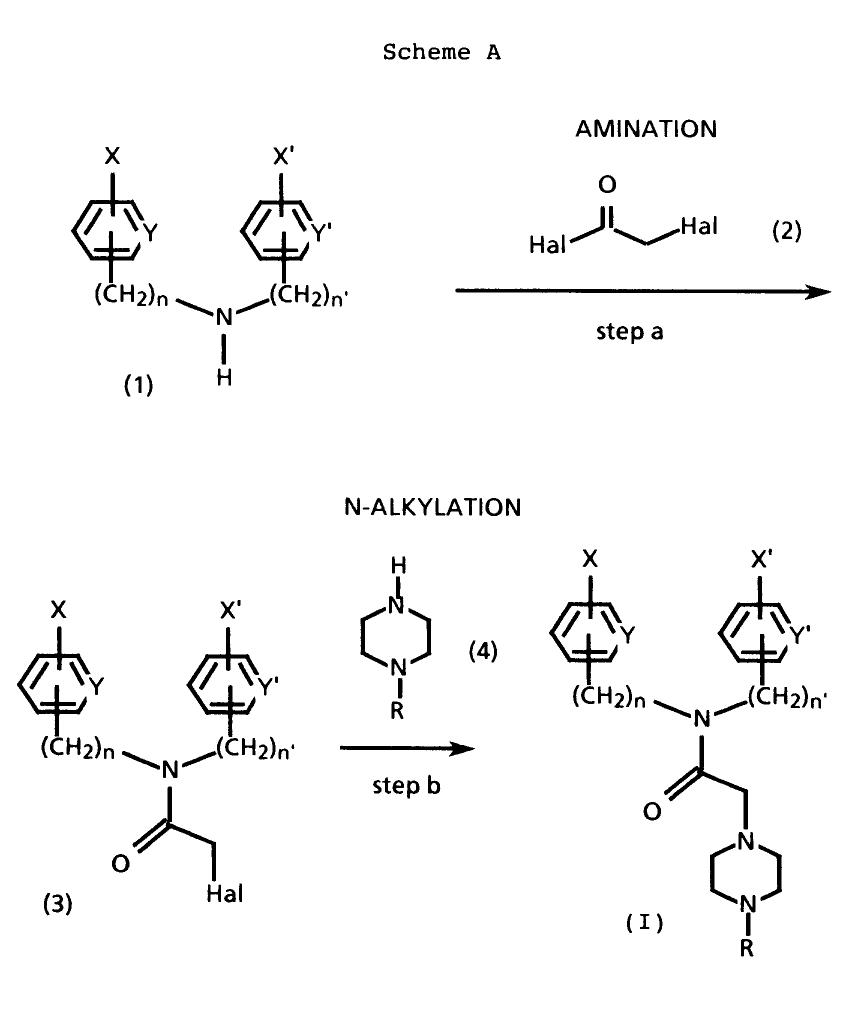
In step a, the appropriate amine of structure (1) is aminated with an appropriate halo acetyl halide of structure (2) to give the corresponding halo acetamide of structure (3).

For example, the appropriate amine of structure (1) is contacted with 1-3 molar equivalents of the appropriate halo acetyl halide of structure (2) in the presence of an appropriate non-nucleophilic base, such as triethylamine, diethylaminopyridine or pyridine. The reactants are typically contacted in a suitable organic solvent such as acetonitrile. The reactants are typically stirred together for a period of time ranging from 10-48 hours and at a temperature range of from 0°C to reflux. The corresponding halo acetamide of structure (3) is recovered from the reaction zone by extractive methods as is known in the art.

In step b, the appropriate halo acetamide of structure (3) is reacted with the appropriate piperazine of structure (4) to give the piperazineacetamide of formula (I).

For example, the appropriate halo acetamide of structure (3) is contacted with 1-4 molar equivalents of the appropriate piperazine of structure (4). The reactants are typically contacted in a suitable organic solvent such as acetonitrile or chloroform. The reactants are typically stirred together for a period of time ranging from 10-48 hours and at a temperature range of from room temperature to reflux. The corresponding piperazineacetamide of formula (I) is recovered from the reaction zone by extractive methods as is known in the art. The piperazineacetamide of formula (I) may be purified by chromatography or converted to an appropriate salt and recrystallized.

The piperazineacetamides of formula (I) wherein X or X' is -(CH₂)mCOOR₁, wherein R₁ is H may be prepared from the corresponding piperazineacetamides of formula (I) wherein X or X' is -(CH₂)mCOOR₁, wherein R₁ is C₁-C₄ alkyl by techniques and procedures well known in the art, such as acid or base hydrolysis.

The piperazineacetamides of formula (I) wherein X or X' is -(CH₂)mNHQ, wherein Q is -C(O)(CH₂)ₚC(O)OR₁ wherein R₁ is hydrogen may be prepared from the corresponding piperazineacetamides of formula (I) wherein X or X' is -(CH₂)mNHQ, wherein Q is -C(O)(CH₂)ₚC(O)OR₁ wherein R₁ is C₁-C₄ alkyl by techniques and procedures well known in the art, such as acid or base hydrolysis.

The piperazineacetamides of formula (I) wherein X or X' is -(CH₂)mNHQ, wherein Q is -C(O)CH=CHC(O)OR₁ wherein R₁ is hydrogen may be prepared from the corresponding piperazineacetamides of formula (I) wherein X or X' is -(CH₂)mNHQ, wherein Q is -C(O)CH=CHC(O)OR₁ wherein R₁ is C₁-C₄ alkyl by techniques and procedures well known in the art, such as acid or base hydrolysis.

The piperazineacetamides of formula (I) wherein X or X' is -(CH₂)mNHQ, wherein Q is -(CH₂)_{p'}C(O)OR₁ wherein R₁ is hydrogen may be prepared from the corresponding piperazineacetamides of formula (I) wherein X or X' is -(CH₂)mNHQ, wherein Q is -(CH₂)_{p'}C(O)OR₁ wherein R₁ is C₁-C₄ alkyl by techniques and procedures well known in the art, such as acid or base hydrolysis.

The piperazineacetamides of formula (I) wherein X or X' is -(CH₂)ₘO(CH₂)_{p'}Z wherein Z is -C(O)OR₁ wherein R₁ is hydrogen may be prepared from the corresponding piperazineacetamides of formula (I) wherein X or X' is -(CH₂)ₘO(CH₂)_{p'}Z, wherein Z is -C(O)OR₁ wherein R₁ is C₁-C₄ alkyl by techniques and procedures well known in the art, such as acid or base hydrolysis.

The piperazineacetamides of formula (I) wherein X or X' is -(CH₂)ₘC(O)OR₁ wherein R₁ is hydrogen may be prepared from the corresponding piperazineacetamides of formula (I) wherein X or X' is -(CH₂)ₘC(O)OR₁ wherein R₁ is C₁-C₄ alkyl by techniques and procedures well known in the art, such as acid or base hydrolysis.

The piperazineacetamides of formula (I) wherein X or X' is -(CH₂)ₘNR₂R₃, wherein R₂ and R₃ are hydrogen may be prepared from the corresponding piperazineacetamides of formula (I) where X or X' is -(CH₂)mNHQ, wherein Q is -(CH₂)_{p'}C(O)OR₁ wherein p' is 0 and R₁ is t-butyl by techniques and procedures well known in the art, such as acid hydrolysis.

The piperazineacetamides of formula (I) wherein R is C₁-C₄ alkyl and X or X' is -(CH₂)mNHQ, wherein Q is -C(O)R₄ may be prepared from the corresponding piperazineacetamides of formula (I) wherein R is C₁-C₄ alkyl and X or X' is -(CH₂)ₘNR₂R₃ wherein R₂ and R₃ are hydrogen by techniques and procedures well known and appreciated by one of ordinary skill in the art, such as such as reaction with the appropriate acid chloride of the structure Cl-C(O)R₄ or an appropriate acid anhydride of the structure R₄-C(O)-O-C(O)-R₄ along with a suitable non-nucleophilic base, such as dimethylaminopyridine, triethylamine or pyridine. For those piperazineacetamides of formula (I) wherein R is hydrogen and X or X' is -(CH₂)ₘNR₂R₃ wherein R₂ and R₃ are hydrogen, the piperazine amino may be protected prior to the acylation reaction. The selection and utilization of suitable protecting groups is well known by one of ordinary skill in the art and is described in "Protective Groups in Organic Syntheses", Theodora W. Greene, Wiley (1981). Subsequent deprotection gives the piperazineacetamides of formula (I) wherein R is hydrogen and X or X' is -(CH₂)mNHQ, wherein Q is -C(O)R₄.

The piperazineacetamides of formula (I) wherein R is C₁-C₄ alkyl and X or X' is -(CH₂)mNHQ, wherein Q is -C(O)(CH₂)ₚC(O)OR₁ wherein R₁ is C₁-C₄ alkyl may be prepared from the corresponding piperazineacetamides of formula (I) wherein R is C₁-C₄ alkyl and X or X' is -(CH₂)ₘNR₂R₃ wherein R₂ and R₃ are hydrogen by techniques and procedures well known and appreciated by one of ordinary skill in the art, such as reaction with the appropriate acid chloride of the structure Cl-C(O)(CH₂)ₚC(O)OR₁ wherein R₁ is C₁-C₄ alkyl along with a suitable non-nucleophilic base, such as dimethylaminopyridine, triethylamine or pyridine. For those piperazineacetamides of formula (I) wherein R is hydrogen and X or X' is -(CH₂)ₘNR₂R₃ wherein R₂ and R₃ are hydrogen, the piperazine amino may be protected prior to the acylation reaction. The selection and utilization of suitable protecting groups is well known by one of ordinary skill in the art and is described in "Protective Groups in Organic Syntheses", Theodora W. Greene, Wiley (1981). Subsequent deprotection gives the piperazineacetamides of formula (I) wherein R is hydrogen and X or X' is -(CH₂)mNHQ, wherein Q is -C(O)(CH₂)ₚC(O)OR₁ wherein R₁ is C₁-C₄ alkyl.

The piperazineacetamides of formula (I) wherein R is C₁-C₄ alkyl and X or X' is -(CH₂)mNHQ, wherein Q is -C(O)CH=CHC(O)OR₁ wherein R₁ is C₁-C₄ alkyl may be prepared from the corresponding piperazineacetamides of formula (I) wherein R is C₁-C₄ alkyl and X or X' is -(CH₂)ₘNR₂R₃ wherein R₂ and R₃ are hydrogen by techniques and procedures well known and appreciated by one of ordinary skill in the art, such as reaction with the appropriate acid chloride of the structure Cl-C(O)CH=CHC(O)OR₁ wherein R₁ is C₁-C₄ alkyl along with a suitable non-nucleophilic base, such as dimethylaminopyridine, triethylamine or pyridine. For those piperazineacetamides of formula (I) wherein R is hydrogen and X or X' is -(CH₂)ₘNR₂R₃ wherein R₂ and R₃ are hydrogen, the piperazine amino may be protected prior to the acylation reaction. The selection and utilization of suitable protecting groups is well known by one of ordinary skill in the art and is described in "Protective Groups in Organic Syntheses", Theodora W. Greene, Wiley (1981). Subsequent deprotection gives the piperazineacetamides of formula (I) wherein R is hydrogen and X or X' is -(CH₂)mNHQ, wherein Q is -C(O)CH=CHC(O)OR₁ wherein R₁ is C₁-C₄ alkyl.

The piperazineacetamides of formula (I)
wherein X or X' is 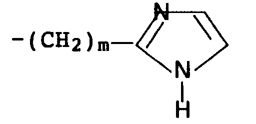
may be prepared from the corresponding piperazineacetamides of formula (I) wherein X or X' is -(CH₂)ₘCN by techniques and procedures well known and appreciated by one of ordinary skill in the art and described in *J.Med.Chem.*, 33, 317 **1990**.

The piperazineacetamides of formula (I)
wherein X or X' is 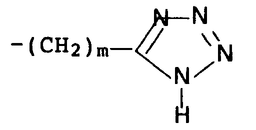
may be prepared from the corresponding piperazineacetamides of formula (I) wherein X or X' is -(CH₂)ₘCN by techniques and procedures well known and appreciated by one of ordinary skill in the art, such as reaction with sodium azide and ammonium chloride.

The piperazineacetamides of formula (I) wherein X or X' is -(CH₂)mO(CH₂)_{p'}Z wherein Z is OH may be prepared from the corresponding piperazineacetamides of formula (I) wherein X or X' is -(CH₂)mO(CH₂)_{p'}Z
wherein Z is 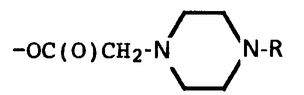
by techniques and procedures well known and appreciated by one of ordinary skill in the art, such as base hydrolysis.

Starting materials for use in Scheme A are readily available to one of ordinary skill in the art.

The following examples present typical syntheses as described in Scheme A. These examples are understood to be illustrative only and are not intended to limit the scope of the present invention in any way. As used herein, the following terms have the indicated meanings: "g" refers to grams; "mmol" refers to millimoles; "mL" refers to milliliters; "bp" refers to boiling point; "mp" refers to melting point; "°C" refers to degrees Celsius; "mm Hg" refers to millimeters of mercury; "µL" refers to microliters; "µg" refers to micrograms; and "µM" refers to micromolar.

### Example 1

### N-(3-Diethylaminomethylphenyl)-N-phenyl-4-methyl-1-piperazineacetamide trioxalate

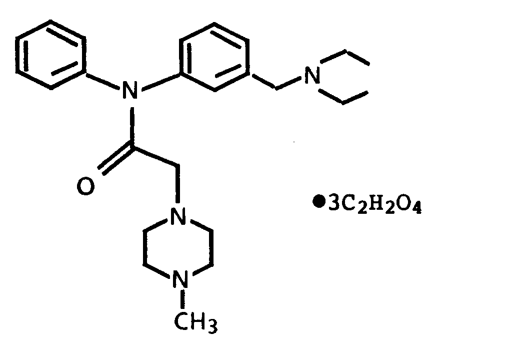

### Step a: N-[3-Diethylaminomethylphenyl]-N-phenylchloroacetamide

Dissolve N-[3-methylphenyl]-N-phenylamine (20g, 0.11mol) in acetonitrile (400mL) and add triethylamine (40mL, 0.33mol). Cool in an ice-bath and add trifluoroacetic anhydride (23mL, 0.165mol). Remove the cooling bath and stir at room temperature for 4 hours under a nitrogen atmosphere. Evaporate the solvent *in vacuo*, partition between saturated aqueous sodium hydrogen carbonate and extract with ethyl acetate. Dry (MgSO₄) and evaporate the solvent *in vacuo* to give N-[3-methylphenyl]-N-phenyltrifluoroacetamide.

¹H NMR (CDCl₃) δ 2.32 (s, 3H), 6.95-7.5 (m, 9H);
¹⁹F NMR δ 94.8 (s).

Dissolve N-[3-methylphenyl]-N-phenyltrifluoroacetamide (4.8g, 1.7mmol) in carbon tetrachloride (250mL) and add N-bromosuccinimide (6g, 3.4mmol). Reflux with a 175W sunlamp for 48 hours under a nitrogen atmosphere. Cool in an ice-bath and remove the precipitate by filtration. Evaporate the solvent *in vacuo* and purify by chromatography (80:20 cyclohexane/ethyl acetate) to give N-[3-bromomethylphenyl]-N-phenyltrifluoroacetamide (5.5g, 91%).

¹H NMR (CDCl₃) δ 4.45 (s, 2H), 7.05-7.65 (m, 9H);
¹⁹F NMR δ 94.8.

Dissolve N-[3-bromomethylphenyl]-N-phenyltrifluoroacetamide (2.18mmol) in excess diethylamine and leave at room temperature for 48 hours. Evaporate the excess diethylamine *in vacuo* to give N-[3-diethylaminomethylphenyl]-N-phenyltrifluoroacetamide.

Mix N-[3-diethylaminomethylphenyl]-N-phenyltrifluoroacetamide (1.74mmol) in 4:1 ethanol/water and add potassium hydroxide (10g). Reflux for 2 days, evaporate the solvent *in vacuo* and take the residue up in water. Extract into ethyl acetate, dry (MgSO₄) and evaporate the solvent *in vacuo* to give N-[3-diethylaminomethylphenyl]-N-phenylamine.

Dissolve N-[3-diethylaminomethylphenyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo* to give the title compound.

### Step b: N-(3-Diethylaminomethylphenyl)-N-phenyl-4-methyl-1-piperazineacetamide trioxalate

Dissolve N-[3-diethylaminomethylphenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and parition between saturated aqueous sodium hydrogen carbonate and ethyl acetate. Separate the organic phase, wash with brine, dry (MgSO₄) and evaporate the solvent *in vacuo* to give N-(3-diethylaminomethylphenyl)-N-phenyl-4-methyl-1-piperazineacetamide.

Dissolve N(3-diethylaminomethylphenyl)-N-phenyl-4-methyl-1-piperazineacetamide (1.5mmol) in tetrahydrofuran (50mL) and add a solution of oxalic acid dihydrate (600mg, 4.5mmol) in tetrahydrofuran (20mL). Filter the precipitate and recrystallize (ethanol/water) to give the title compound.

### Example 2

### N,N-Diphenyl-4-methyl-1-piperazineacetamide dioxalate

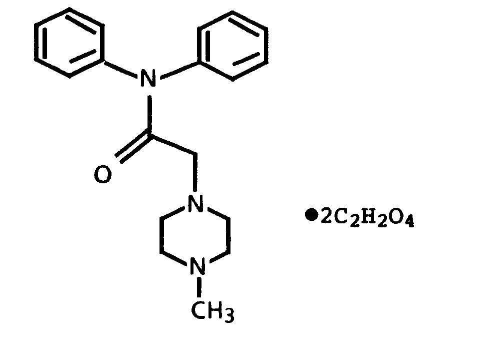

### Step a: N,N-Diphenylchloroacetamide

Dissolve diphenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo* to give the title compound.

### Step b: N,N-Diphenyl-4-methyl-1-piperazineacetamide dioxalate

Dissolve N,N-diphenylchloroacetamide (30mmol) in acetonitrile (150mL), add N-methylpiperazine (12g, 120mmol) and reflux overnight under a nitrogen atmosphere. Evaporate the solvent *in vacuo* and parition between saturated aqueous sodium hydrogen carbonate and ethyl acetate. Separate the organic phase, wash with brine, dry (MgSO₄) and evaporate the solvent *in vacuo* to give N,N-diphenyl-4-methyl-1-piperazineacetamide.

Dissolve N,N-diphenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize (isopropanol/water) to give the title compound.

### Example 3

### N-[4-(t-Butoxycarbamoyl)phenyl]-N-phenyl-4-methyl-1-piperazineacetamide dioxalate

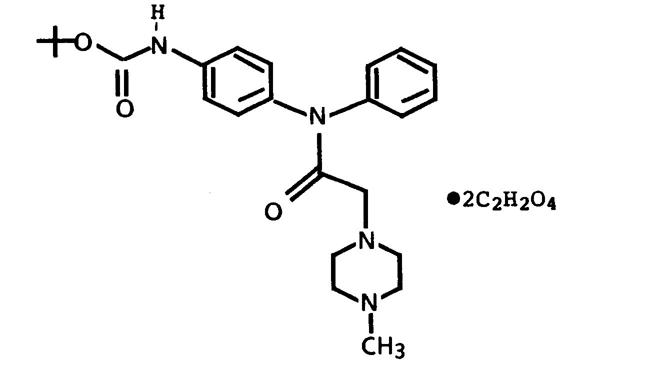

### Step a: N-[4-t-Butoxycarbamoylphenyl]-N-phenylchloroacetamide

Dissolve 4-aminodiphenylamine (10g, 54mmol) in chloroform (100mL), add triethylamine (11mL, 81mmol) and cool in an ice-bath. Slowly add a solution of di-tert-butydicarbonate (12g, 54mmol) in chloroform (50mL), remove the ice-bath and stir at room temperature overnight under a nitrogen atmosphere. Evaporate the solvent *in vacuo* and purify by chromatography (80:20 cyclohexane/ethyl acetate) to give N-[4-t-Butoxycarbamoylphenyl]-N-phenylamine (12g, 80%).

¹H NMR (CDCl₃) δ 1.5 (s, 9H), 5.6 (S, 1H), 6.45 (S, 1H), 6.8-7.35 (m, 9H).

Dissolve N-[4-t-Butoxycarbamoylphenyl]-N-phenylamine (11g, 38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography (80:20 cyclohexane/ethyl acetate) to give the title compound (11g, 84%).

¹H NMR (CDCl₃) δ 1.5 (s, 9H), 4 (s, 2H), 6.7 (s, 1H), 7.1-7.5 (m, 9H).

### Step b: N[4-(t-Butoxycarbamoyl)phenyl]-N-phenyl-4-methyl-1-piperazineacetamide dioxalate

Dissolve N-[4-t-Butoxycarbamoylphenyl]-N-phenylchloroacetamide (11g, 30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N[4-(t-Butoxycarbamoyl)phenyl]-N-phenyl-4-methyl-1-piperazineacetamide (12g, 94%).

¹H NMR (CDCl₃) δ 1.5 (s, 9H), 2.25 (s, 3H), 2.8-2.8 (m, 8H), 3.05 (s, 2H), 6.6 (s, 1H), 7.05-7.5 (m, 9H).

Dissolve N[4-(t-Butoxycarbamoyl)phenyl]-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize (isopropanol/water) to give the title compound.

### Example 4

### N-(4-Aminophenyl)-N-phenyl-4-methyl-1-piperazineacetamide dimaleate

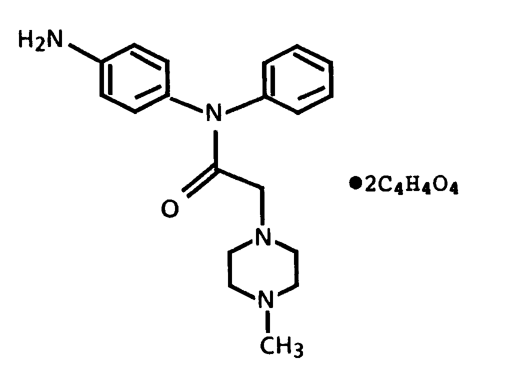

### Method A:

Dissolve N[4-(t-Butoxycarbamoyl)phenyl]-N-phenyl-4-methyl-1-piperazineacetamide (6g, 14mmol) in a 40:60 mixture of methylene chloride/trifluoroacetic acid (200mL), cool in an ice-bath and stir for 1 hour. Evaporate the solvent *in vacuo* and partition between saturated aqueous sodium hydrogen carbonate and ethyl acetate. Separate the organic phase, dry (MgSO₄) and evaporate the solvent *in vacuo* to give N(4-aminophenyl)-N-phenyl-4-methyl-1-piperazineacetamide (3.5g, 78%).

¹H NMR (CDCl₃) δ 2.4 (s, 3H), 2.5-2.85 (m, 8H), 3.1 (s, 2H), 3.4-4 (bs, 2H), 6.65 (d, 2H), 7.03 (d, 2H), 7.1-7.5 (m, 5H).

Dissolve N-4-aminophenyl-N-phenyl-4-methyl-1-piperazineacetamide (500mg, 1.5mmol) in tetrahydrofuran (50mL) and add a solution of maleic acid (348mg, 3mmol) in tetrahydrofuran (20mL). Filter the precipitate and recrystallize (isopropanol/water) to give the title compound.

### Method B:

### Step a: N-[4-Nitrophenyl]-N-phenylchloroacetamide

Dissolve 4-nitrodiphenylamine (38mmol) in toluene (450mL) and add chloroacetyl chloride (4.5mL, 57mmol). Reflux the mixture overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo* to give the title compound.

### Step b: N-4-Aminophenyl-N-phenyl-4-methyl-1-piperazineacetamide dimaleate

Dissolve N-[4-nitrophenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate.

Separate the organic phase, wash with brine, dry (MgSO₄) and evaporate the solvent *in vacuo* to give N-4-nitrophenyl-N-phenyl-4-methyl-1-piperazineacetamide.

Dissolve N-4-nitrophenyl-N-phenyl-4-methyl-1-piperazineacetamide (12mmol) in ethanol (100mL) and add tin(II) chloride dihydrate (16.3g). Warm to between 65°C and 75°C for 4 to 5 hours. Cool to room temperature and pour into a mixture of ethyl acetate (300mL) and water (200mL). Add solid potassium carbonate and stir occasionally until the carbon dioxide evolution ceases. Filter through filter aid and separate the organic phase. Dry (MgSO₄) and evaporate the solvent *in vacuo* to give N-4-aminophenyl-N-phenyl-4-methyl-1-piperazineacetamide.

Dissolve N-4-aminophenyl-N-phenyl-4-methyl-1-piperazineacetamide (500mg, 1.5mmol) in tetrahydrofuran (50mL) and add a solution of maleic acid (348mg, 3mmol) in tetrahydrofuran (20mL). Filter the precipitate and recrystallize (isopropanol/water) to give the title compound.

### Example 5

### N-4-Acetamidophenyl-N-phenyl-4-methyl-1-piperazineacetamide dioxalate

Mix N-4-aminophenyl-N-phenyl-4-methyl-1-piperazineacetamide (200mg, 0.6mmol), triethylamine (0.17mL), acetyl chloride (0.045mL), dimethylaminopyridine (5mg) and acetonitrile (10mL). Stir at room temperature for 24 hours, evaporate the solvent in vacuo and partition between saturated aqueous sodium hydrogen carbonate and ethyl acetate. Separate the organic phase, dry (MgSO₄) and evaporate the solvent *in vacuo* to give N-4-acetamidophenyl-N-phenyl-4-methyl-1-piperazineacetamide. 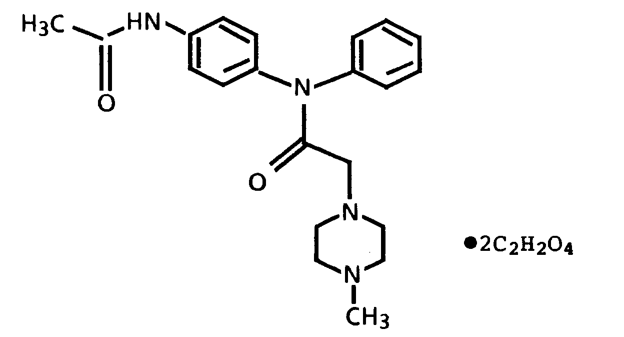
Dissolve N-4-acetamidophenyl-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize (isopropanol/water) to give the title compound.

### Example 6

### N-2-(t-Butoxycarbamoyl)phenyl-N-phenyl-4-methyl-1-piperazineacetamide dioxalate

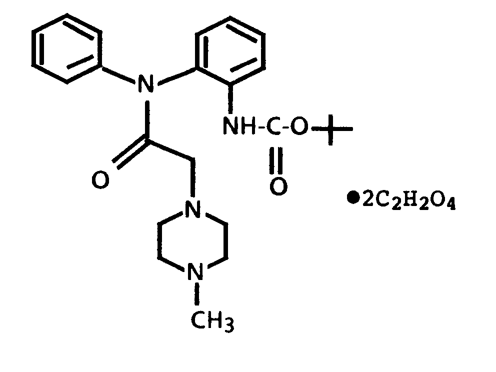

Prepare as described above in Example 3 for N-4-(t-Butoxycarbamoyl)phenyl-N-phenyl-4-methyl-1- piperazineacetamide dioxalate but using 2-aminodiphenylamine instead of 4-aminodiphenylamine.

### Example 7

### N-3-Hydroxyphenyl-N-phenyl-4-methyl-1-piperazineacetamide dioxalate

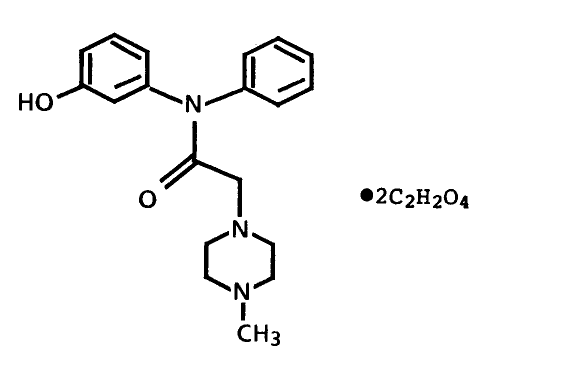

Prepare as described above in Example 2 for N,N-diphenyl-4-methyl-1-piperazineacetamide dioxalate but using N-[3-hydroxyphenyl]-phenylamine instead of diphenylamine and using 3 equivalents of chloro acetyl chloride, 3 equivalents of pyridine and 4 equivalents of N-methyl-piperazine (see Example 9).

### Example 8

### N-3-Methylphenyl-N-phenyl-4-methyl-1-piperazineacetamide dioxalate

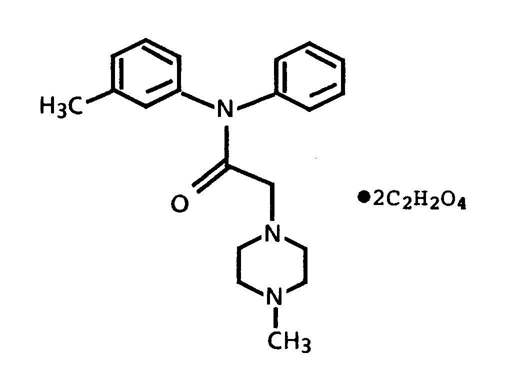

Prepare as described above in Example 2 for N,N-diphenyl-4-methyl-1-piperazineacetamide dioxalate but using N-[3-methylphenyl]-phenylamine instead of diphenylamine.

### Example 9

### N-3-[(4-Methyl-1-piperazinomethyl)carboxymethyl]phenyl-N-phenyl-4-methyl-1-piperazineacetamide tetraoxalate

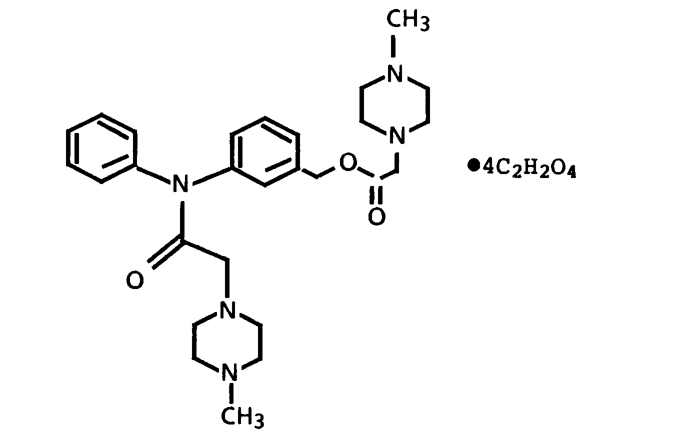

### Step a: N-[3-[chloromethyl)carboxymethyl]phenyl]-N-phenylchloroacetamide

Mix N-[3-bromomethylphenyl]-N-phenyltrifluoroacetamide (16g, 44mmol), potassium acetate (22g) and dimethylformamide (200mL). Heat to 50°C for 48 hours, add water (200mL) and separate the aqueous phase. Extract the aqueous phase with a mixture of 1:1 ethyl ether/cyclohexane, dry (MgSO₄) and evaporate the solvent *in vacuo* to give N-[3-acetoxymethylphenyl]-N-phenyltrifluoroacetamide.

Mix N-[3-acetoxymethylphenyl]-N-phenyltrifluoroacetamide (44mmol), potassium carbonate (20g) and a mixture of 1:4 water/methanol (300mL). Stir at room temperature for 24 hours, evaporate the solvent *in vacuo* and partition between water and ethyl ether. Separate the organic phase, dry (MgSO₄) and evaporate the solvent *in vacuo*. Purify by chromatography (2:3 ethyl acetate/cyclohexane) to give N-[3-hydroxymethylphenyl]-N-phenylamine (7.6g).

Dissolve N-[3-hydroxymethylphenyl]-N-phenylamine (38mmol) in acetonitrile (450mL) and add pyridine (9.2mL, 114mmol). Cool in an ice-bath and slowly add chloroacetyl chloride (9mL, 114mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo* to give the title compound.

### Step b: N-3-[(4-Methyl-1-piperazinomethyl)carboxymethyl]-phenyl-N-phenyl-4-methyl-1-piperazineacetamide tetraoxalate

Dissolve N-[3-[(chloromethyl)carboxymethyl]phenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-3-[(4-methyl-1-piperazinomethyl)carboxy-methyl]phenyl-N-phenyl-4-methyl-1-piperazineacetamide.

Dissolve N-3-[(4-methyl-1-piperazinomethyl)carboxy-methyl]phenyl-N-phenyl-4-methyl-1-piperazineacetamide (1.5mmol) in tetrahydrofuran (50mL) and add a solution of oxalic acid dihydrate (800mg, 6mmol) in tetrahydrofuran (15mL). Filter the precipitate and recrystallize (ethanol/water) to give the title compound.

### Example 10

### N-3-Hydroxymethylphenyl-N-phenyl-4-methyl-1-piperazineacetamide dioxalate

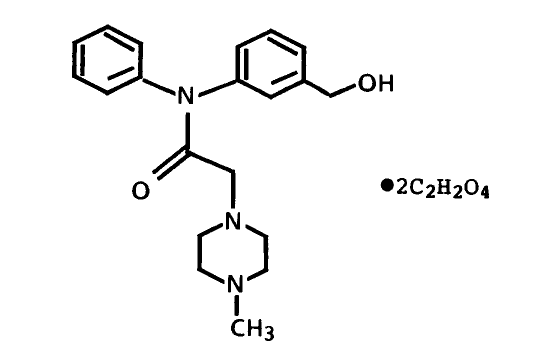

Mix N-3-[(4-methyl-1-piperazinomethyl)carboxymethyl]phenyl-N-phenyl-4-methyl-1-piperazineacetamide (400mg, 0.48mmol), potassium carbonate (2g) and a mixture of 4:1 methanol/water. Stir at room temperature for 24 hours, partition between saturated aqueous sodium hydrogen carbonate and ethyl acetate. Separate the organic phase, dry (MgSO₄) and evaporate the solvent *in vacuo* to give N-3-hydroxymethylphenyl-N-phenyl-4-methyl-1-piperazineacetamide.

Dissolve N-3-hydroxymethylphenyl-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize (ethanol) to give the title compound.

### Example 11

### N-3-(1-Imidazoylmethyl)phenyl-N-phenyl-4-methyl-1-piperazineacetamide dioxalate

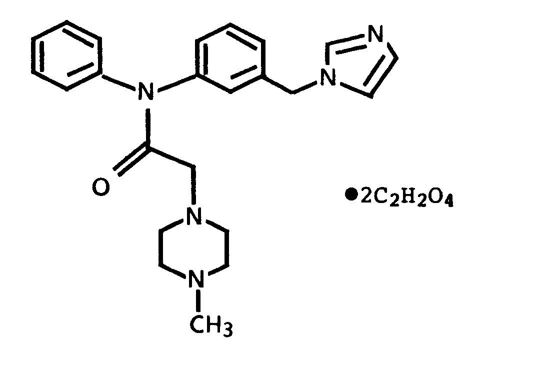

### Step a: N-[3-(1-Imidazoylmethyl)phenyl]-N-phenylchloroacetamide

Mix N-[3-bromomethylphenyl]-N-phenyltrifluoroacetamide (1g), imidazole (1g) and acetonitrile (10mL). Reflux for 3 days, evaporate the solvent *in vacuo* and take the residue up in water. Add sodium hydrogen carbonate and extract into ethyl acetate. Purify by chromatography (8:2 ethyl acetate/methanol) to give N-[3-(1-imidazoylmethyl)phenyl]-N-phenyltrifluoroacetamide.

Mix N-[3-(1-imidazoylmethyl)phenyl]-N-phenyltrifluoroacetamide (600mg) in 4:1 ethanol/water and add potassium hydroxide (10g). Reflux for 2 days, evaporate the solvent *in vacuo* and take up the residue in water. Extract into ethyl acetate, dry (MgSO₄) and evaporate the solvent *in vacuo* to give N-[3-(1-imidazoylmethyl)phenyl]-N-phenylamine.

Dissolve N-[3-(1-imidazoylmethyl)phenyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo* to give the title compound.

### Step b: N-3-(1-Imidazoylmethyl)phenyl-N-phenyl-4-methyl-1-piperazineacetamide dioxalate

Dissolve N-[3-(1-imidazoylmethyl)phenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo*. Purify by chromatography to give N-3-(1-imidazoylmethyl)phenyl-N-phenyl-4-methyl-1-piperazineacetamide.

Dissolve N-3-(1-imidazoylmethyl)phenyl-N-phenyl-4-methyl-1-piperazineacetamide (1.5mmol) in tetrahydrofuran (50mL) and add a solution of oxalic acid dihydrate (400mg, 3mmol) in tetrahydrofuran (20mL). Filter the precipitate and recrystallize to give the title compound.

### Example 12

### N-4-Cyanophenyl-N-phenyl-4-methyl-1-piperazineacetamide dioxalate

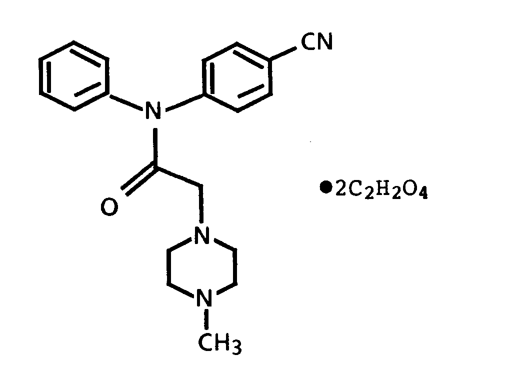

### Step a: N-[4-Cyanophenyl]-N-phenylchloroacetamide

Dissolve cuprous cyanide (50mmol) in cold water (30mL) and cool to 0-5°C by the addition of ice. Pour toluene (10mL) on the surface and add slowly to a cold solution of 4-diazodiphenylamine sulfate (40mmol) in water (50mL). Stir at 0-5°C for 30 minutes and then at room temperature for 3 hours. Separate the organic phase and extract the aqueous phase with toluene. Combine the organic phases, dry (MgSO₄) and evaporate the solvent *in vacuo* to give N-[4-cyanophenyl]-N-phenylamine.

Dissolve N-[4-cyanophenyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo* to give the title compound.

### Step b: 4-Cyano-N,N-diphenyl-4-methyl-1-piperazineacetamide trioxalate

Dissolve N-[4-cyanophenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo*. Purify by chromatography to give N-4-cyanophenyl-N-phenyl-4-methyl-1-piperazineacetamide.

Dissolve N-4-cyanophenyl-N-phenyl-4-methyl-1-piperazineacetamide (1.5mmol) in tetrahydrofuran (50mL) and add a solution of oxalic acid dihydrate (400mg, 3mmol) in tetrahydrofuran (20mL). Filter the precipitate and recrystallize (isopropanol/water) to give the title compound.

### Example 13

### N-(3-Methoxycarbonylmethyl)phenyl-N-phenyl-4-methyl-1-piperazineacetamide dioxalate

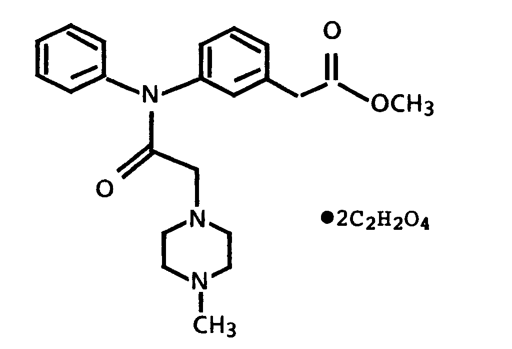

### Step a: N-[Methoxycarbonylmethylphenyl]-N-phenylchloroacetamide

Dissolve N-[3-bromomethylphenyl]-N-phenyltrifluoroacetamide (41.3mmol) in 95% ethanol (40mL) and heat to reflux. Add, in portions through the reflux condenser, a solution of potassium cyanide (4.3g) in water (6mL). Add additional water (4mL) to effect the transfer. Reflux for 9 hours, pour into water and extract into ethyl ether. Wash with brine, dry (MgSO₄) and evaporate the solvent *in vacuo* to give N-[3-cyanomethylphenyl]-N-phenyltrifluoroacetamide.

Mix N-[3-cyanomethylphenyl]-N-phenyltrifluoroacetamide (1.3g) and concentrated hydrochloric acid (100mL) and reflux overnight. Dilute with 4:1 ethanol/water and carefully add potassium hydroxide (10g). Reflux for 2 days, evaporate the solvent *in vacuo* and take up the residue in water. Extract into ethyl acetate, Dry (MgSO₄) and evaporate the solvent *in vacuo* to give N-[3-carboxymethylphenyl]-N-phenylamine.

Dissolve N-(3-Carboxymethylphenyl)-N-phenylamine in a MeOH and trimethoxymethane (1/1) mixture. HCl is bubbled through the mixture for 10 minutes and the mixture is refluxed overnight. The solvent is removed *in vacuo*, to afford N-(3-methoxycarbonylmethylphenyl)-N-phenylamine.

Dissolve N-[3-methoxycarbonylmethylphenyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solven*t in vacuo* and partition between saturated aqueous sodium hydrogen carbonate and ethyl acetate. Separate the organic phase, dry (MgSO₄) and evaporate the solvent *in vacuo* to give the title compound.

### Step b: N-3-Methoxycarbonylmethylphenyl-N-phenyl-4-methyl-1-piperazineacetamide dioxalate

Dissolve N-[methoxycarbonylmethylphenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo*. Purify by chromatography to give N-3-methoxycarbonylmethylphenyl-N-phenyl-4-methyl-1-piperazineacetamide.

Dissolve N-3-methoxycarbonylmethylphenyl-N-phenyl-4-methyl-1-piperazineacetamide (1.5mmol) in tetrahydrofuran (50mL) and add a solution of oxalic acid dihydrate (400mg, 3mmol) in tetrahydrofuran (20mL). Filter the precipitate and recrystallize to give the title compound.

### Example 14

### N-3-Carboxymethylphenyl-N-phenyl-4-methyl-1-piperazineacetamide dihydrochloride

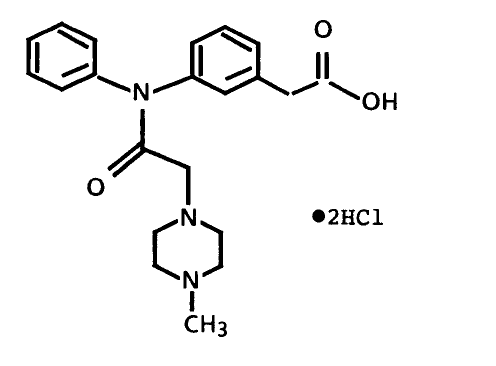

Dissolve N-3-methoxycarbonylmethylphenyl-N-phenyl-4-methyl-1-piperazineacetamide (500mg) in 0.1N aqueous hydrochloric acid. Reflux for 4 hours, remove the water *in vacuo* and recrystallize (isopropanol) to give the title compound.

### Example 15

### N-3-Dimethylaminocarbonylmethylphenyl-N-phenyl-4-methyl-1-piperazineacetamide dioxalate

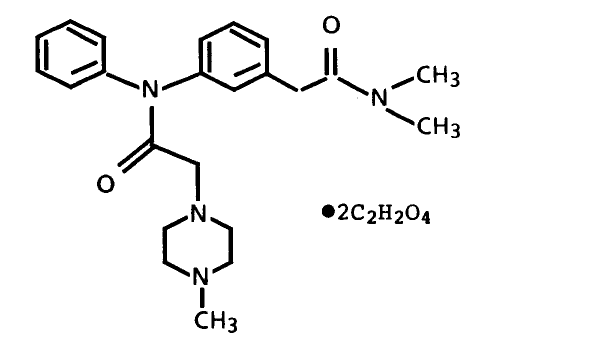

Mix 3-carboxymethyl-N,N-diphenylamine (0.34mmol), methylene chloride (120mL) and dimethylformamide (0.05mL) under a nitrogen atmosphere. Add oxalyl chloride (3.5mL, 40mmol) over about 5 minutes with stirring. Stir at ambient temperature for 3 hours. Cool the mixture in an ice bath and bubble dimethylamine into the solution for 10 minutes. Stir the mixture for 1 hour and evaporate the solvent *in vacuo*. Partition the residue between saturated aqueous sodium hydrogen carbonate and ethyl acetate. Separate the organic phase, wash with brine, dry (MgSO₄) and evaporate the solvent *in vacuo* to give 3-dimethylaminocarbonylmethyl-diphenylamine.

The title compound is prepared as described in Example 2 for N,N-diphenyl-4-methyl-1-piperazineacetamide dioxalate but using 3-dimethylaminocarbonylmethyl-diphenylamine instead of diphenylamine.

### Example 16

### N-4-(t-Butoxycarbamoyl)phenyl-N-4'-methoxyphenyl-4-methyl-1-piperazineacetamide dioxalate

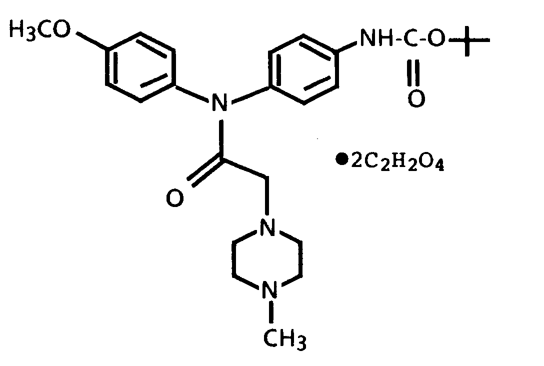

Prepare as described above in Example 3 for N-4-(t-Butoxycarbamoyl)phenyl-N-phenyl-4-methyl-1-piperazineacetamide dioxalate but using N-[4-aminophenyl]-N-[4'-methoxyphenyl]amine instead of 4-aminodiphenylamine.

### Example 17

### N-4-Aminophenyl-N-4'-methoxyphenyl-4-methyl-1-piperazineacetamide sesqui oxalate

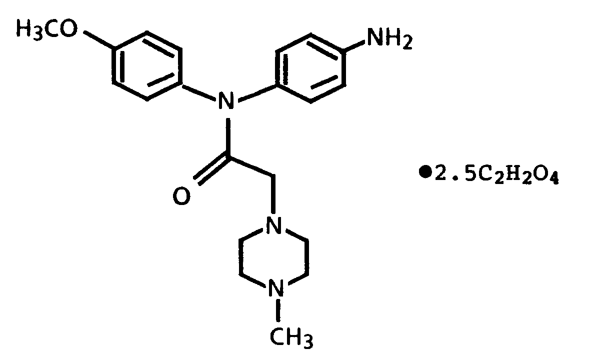

Prepare as described above in Example 4 for N-4-aminophenyl-N-phenyl-4-methyl-1-piperazineacetamide dioxalate but using N-4-(t-Butoxycarbamoyl)phenyl-N-4'-methoxyphenyl-4-methyl-1-piperazineacetamide instead of N-4-(t-Butoxycarbamoyl)phenyl-N-phenyl-4-methyl-1-piperazineacetamide.

### Example 18

### N-4-Methylaminophenyl-N-phenyl-4-methyl-1-piperazineacetamide dioxalate

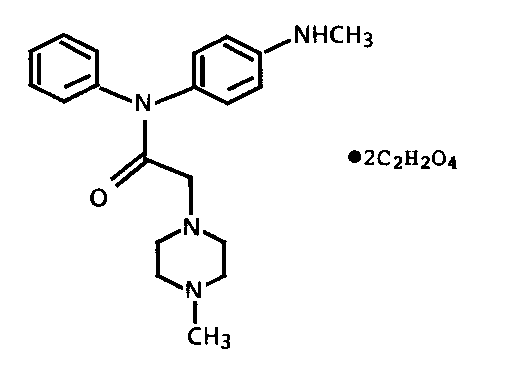

### Step a: N-[4-(N'-methyl-t-Butoxycarbamoyl)phenyl]-N-phenylchloroacetamide

Suspend lithium aluminum hydride (170mg) in tetrahydrofuran and add, by dropwise addition, a solution of N-[4-t-Butoxycarbamoylphenyl]-N-phenylamine (500mg) in tetrahydrofuran (20mL). Reflux for 2 hour, cool to 0°C and add Na₂SO₄·10H₂O and filter aid until no further reaction is observed. Stir the mixture overnight, filter through filter aid and evaporate the solvent *in vacuo* to give N-[4-methylaminophenyl]-N-phenylamine.

Dissolve N-[4-methylaminophenyl]-N-phenylamine (54mmol) in chloroform (100mL), add triethylamine (11mL, 81mmol) and cool in an ice-bath. Slowly add a solution of di-tert-butydicarbonate (12g, 54mmol) in chloroform (50mL), remove the ice-bath and stir at room temperature overnight under a nitrogen atmosphere. Evaporate the solvent in vacuo and purify by chromatography (80:20 cyclohexane/ethyl acetate) to give N-[4-(N'-methyl-t-Butoxycarbamoyl)phenyl]-N-phenylamine.

Dissolve N-[4-(N'-methyl-t-Butoxycarbamoyl)phenyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography to give the title compound.

### Step b: N-4-Methylaminophenyl-N-phenyl-4-methyl-1-piperazineacetamide dioxalate

Dissolve N-[4-(N'-methyl-t-Butoxycarbamoyl)phenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-4-(N'-methyl-t-butoxycarbamoyl)phenyl-N-phenyl-4-methyl-1-piperazineacetamide.

Dissolve N-4-(N'-methyl-t-Butoxycarbamoyl)phenyl-N-phenyl-4-methyl-1-piperazineacetamide (14mmol) in a 40:60 mixture of methylene chloride/trifluoroacetic acid (200mL), cool in an ice-bath and stir for 1 hour. Evaporate the solvent *in vacuo* and partition between saturated aqueous sodium hydrogen carbonate and ethyl acetate. Separate the organic phase, dry (MgSO₄) and evaporate the solvent *in vacuo* to give N-4-methylaminophenyl-N-phenyl-4-methyl-1-piperazineacetamide.

Dissolve N-4-methylaminophenyl-N-phenyl-4-methyl-1-piperazineacetamide (1.5mmol) in tetrahydrofuran (50mL) and add a solution of oxalic acid dihydrate (400mg, 3mmol) in tetrahydrofuran (20mL). Filter the precipitate and recrystallize to give the title compound.

### Example 19

### N-4-Dimethylaminophenyl-N-phenyl-4-methyl-1-piperazineacetamide dioxalate

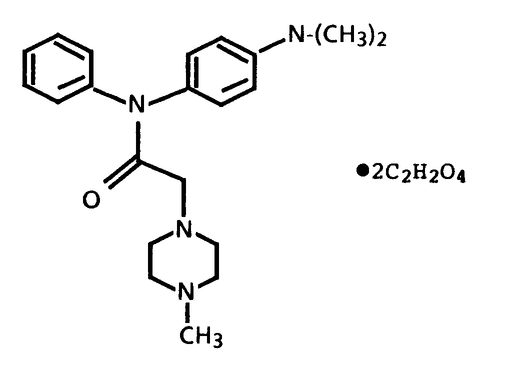

### Step a: N-[4-Dimethylaminophenyl]-N-phenylchloroacetamide

Mix N-[4-methylaminophenyl]-N-phenylamine (100mg, 0.5mmol) and formic acid (1mL) and reflux for 5 hours with azeotropic removal of the formed water. Evaporate the solvent *in vacuo* to give N-[4-N'-methylformamidophenyl]-N-phenylamine.

Suspend lithium aluminum hydride (35mg) in tetrahydrofuran and add, by dropwise addition, a solution of N-[4-N'-methylformamidophenyl]-N-phenylamine (100mg) in tetrahydrofuran. Reflux for 3 hours, add Na₂SO₄·10H₂O and filter aid until no further reaction is observed. Stir the mixture overnight, filter through filter aid and evaporate the solvent *in vacuo* to give N-[4-dimethylaminophenyl]-N-phenylamine.

Dissolve N-[4-dimethylaminophenyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography to give the title compound.

### Step b: N-4-Dimethylaminophenyl-N-phenyl-4-methyl-1-piperazineacetamide dioxalate

Dissolve N-[4-dimethylaminophenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-4-dimethylaminophenyl-N-phenyl-4-methyl-1-piperazineacetamide.

Dissolve N-4-dimethylaminophenyl-N-phenyl-4-methyl-1-piperazineacetamide (1.5mmol) in tetrahydrofuran (50mL) and add a solution of oxalic acid dihydrate (400mg, 3mmol) in tetrahydrofuran (20mL). Filter the precipitate and recrystallize to give the title compound.

### Example 20

### N-Phenyl-N-(2-pyridyl)-4-methyl-1-piperazineacetamide dioxalate

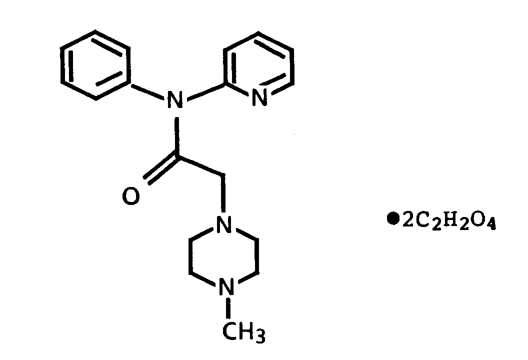

Prepare as described above in Example 2 for N,N-diphenyl-4-methyl-1-piperazineacetamide dioxalate but using N-phenyl-N-(2-pyridyl)amine instead of diphenylamine.

### Example 21

### N-3-Trifluoromethyl-phenyl-N-(2-pyridyl)-4-methyl-1-piperazineacetamide dioxalate

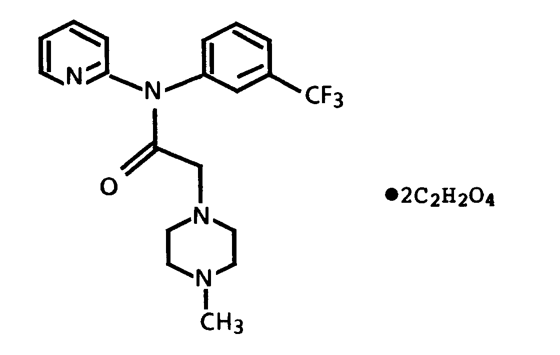

Reduce N-nitroso-N-(2-pyridyl)-3-(trifluoromethyl)aniline with Raney-nickel essentially as described in *Organic Synthesis* (VI), 542 **1988** to give N-3-trifluoromethylphenyl-N-(2-pyridyl)amine.

Prepare the title compound as described above in Example 2 for N,N-diphenyl-4-methyl-1-piperazineacetamide dioxalate but using N-3-trifluoromethylphenyl-N-(2-pyridyl)amine instead of diphenylamine.

### Example 22

### N,N-di[4-(t-Butoxycarbamoyl)phenyl]-4-methyl-1-piperazineacetamide dimaleate

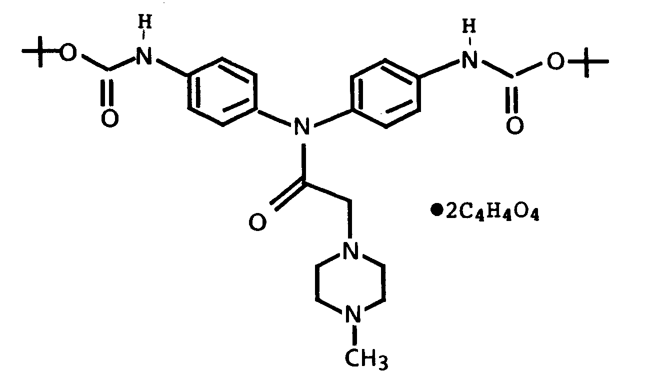

### Step a: N,N-di[4-t-Butoxycarbamoylphenyl]chloroacetamide

Dissolve 4,4'-diaminodiphenylamine (54mmol) in chloroform (100mL), add triethylamine (22ml, 162mmol) and cool in an ice-bath. Slowly add a solution of di-tert-butyldicarbonate (24g, 108mmol) in chloroform (100mL), remove the ice-bath and stir at room temperature overnight under a nitrogen atmosphere. Evaporate the solvent *in vacuo* and purify by chromatography to give N,N-[4,4'-di-t-Butoxycarbamoyldiphenylamine.

Dissolve N,N-[4,4'-di-t-Butoxycarbamoyldiphenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo.* Purify by chromatography to give the title compound.

### Step b: N,N-di[4-(t-Butoxycarbamoyl)phenyl]-4-methyl-1-piperazineacetamide dimaleate

Dissolve N,N-di[4-t-Butoxycarbamoylphenyl]chloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N,N-di [4-(t-Butoxycarbamoyl)phenyl]-4-methyl-1-piperazineacetamide.

Dissolve N,N-di[4-(t-Butoxycarbamoyl)phenyl]-4-methyl-1-piperazineacetamide (1.5mmol) in tetrahydrofuran (50mL) and add a solution of maleic acid (350mg, 3mmol) in tetrahydrofuran (15mL). Filter the precipitate and recrystallize to give the title compound.

### Example 23

### N,N-di[4-aminophenyl-4-methyl-1-piperazineacetamide dimaleate

Dissolve N,N-di[4-(t-Butoxycarbamoyl)phenyl]-4-methyl-1-piperazineacetamide (14mmol) in a 40:60 mixture of methylene chloride/trifluoroacetic acid (200mL), cool in an ice-bath and stir for 1 hour. Evaporate the solvent *in vacuo* and partition between saturated aqueous sodium hydrogen carbonate and ethyl acetate. Separate the organic phase, dry (MgSO₄) and evaporate the solvent *in vacuo* to give N,N-di[4-aminophenyl]-4-methyl-1-piperazineacetamide.

Dissolve 4,4'-di[aminophenyl]-4-methyl-1-piperazineacetamide (1.5mmol) in tetrahydrofuran (50mL) and add a solution of 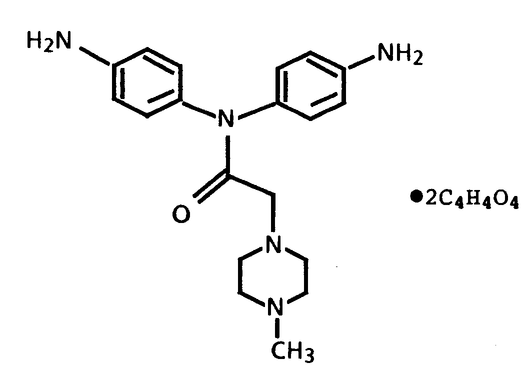
maleic acid (350mg, 3mmol) in tetrahydrofuran (20mL). Filter the precipitate and recrystallize (ethanol) to give the title compound.

### Example 24

### N-4-(t-Butoxycarbamoyl)phenyl-N-phenyl-1-piperazineacetamide dioxalate

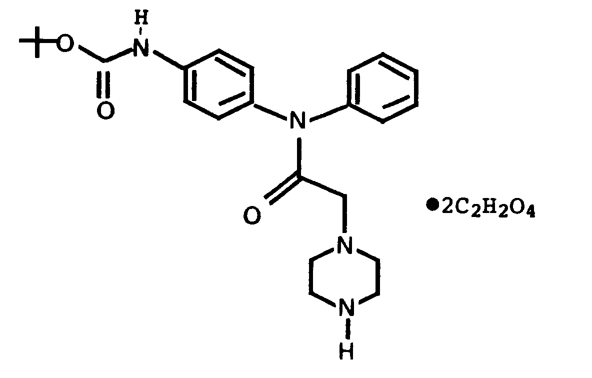

### Step b: N-4-(t-Butoxycarbamoyl)phenyl-N-phenyl-1-piperazineacetamide dioxalate

Dissolve N-[4-t-Butoxycarbamoylphenyl]-N-phenylchloroacetamide (11g, 30mmol) in acetonitrile (150mL), add piperazine (120mmol) and reflux overnight under a nitrogen atmosphere. Evaporate the solvent *in vacuo* and parition between saturated aqueous sodium hydrogen carbonate and ethyl acetate. Separate the organic phase, wash with brine, dry (MgSO₄) and evaporate the solvent *in vacuo* to give N-4-(t-Butoxycarbamoyl)phenyl-N-phenyl-1-piperazineacetamide.

Dissolve N-4-(t-Butoxycarbamoyl)phenyl-N-phenyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize to give the title compound.

### Example 25

### N-4-Aminophenyl-N-phenyl-1-piperazineacetamide dimaleate

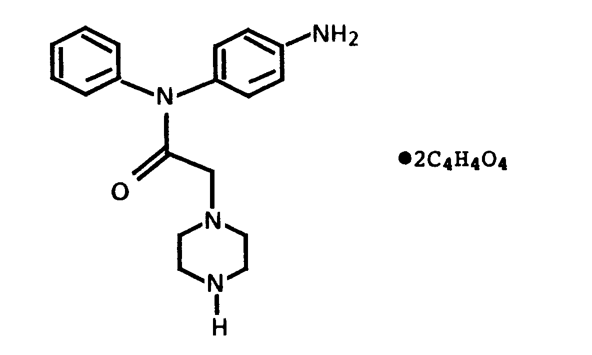

Dissolve N-4-(t-Butoxycarbamoyl)phenyl-N-phenyl-1-piperazineacetamide (14mmol) in a 40:60 mixture of methylene chloride/trifluoroacetic acid (200mL), cool in an ice-bath and stir for 1 hour. Evaporate the solvent *in vacuo* and partition between saturated aqueous sodium hydrogen carbonate and ethyl acetate. Separate the organic phase, dry (MgSO₄) and evaporate the solvent *in vacuo* to give N-4-aminophenyl-N-phenyl-1-piperazineacetamide.

Dissolve N-4-aminophenyl-N-phenyl-1-piperazineacetamide (500mg, 1.5mmol) in tetrahydrofuran (50mL) and add a solution of maleic acid (350mg, 3mmol) in tetrahydrofuran (20mL). Filter the precipitate and recrystallize to give the title compound.

### Example 26

### N,N-di[4-aminobenzyl]-4-methyl-1-piperazineacetamide tetraoxalate

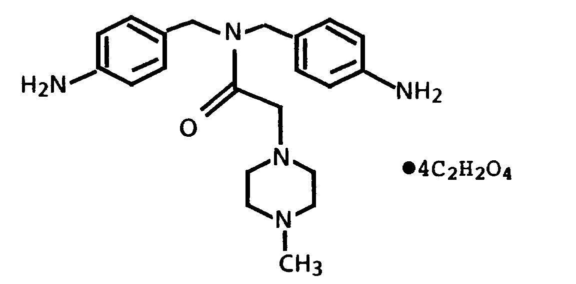

Prepare as described above in Example 2 for N,N-diphenyl-4-methyl-1-piperazineacetamide but using 4,4'-diaminodibenzylamine instead of N,N-diphenylamine and using 1 equivalent of chloroacetylchloride and 1 equivalent of pyridine at 0°C.

### Example 27

### N,N-di[4-(t-Butoxycarbamoyl)benzyl]-4-methyl-1-piperazineacetamide dioxalate

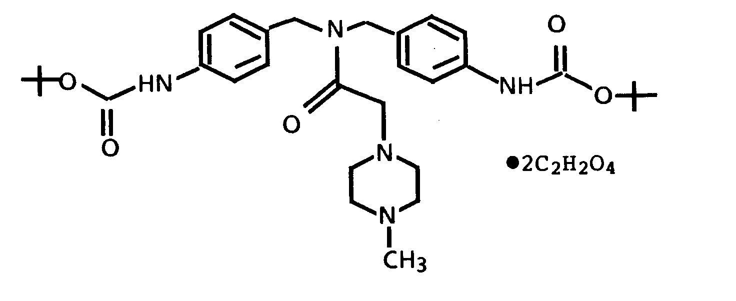

Dissolve N,N-di[4-aminobenzyl]-4-methyl-1-piperazineacetamide (2.27mmol) in anhydrous tetrahydrofuran (15mL). Treat with pyridine (4.54mmol) followed by di-tert-butyl dicarbonate (4.54mmol) and stir at ambient temperature overnight. Partition between ethyl ether and water. Separate the organic phase, dry (MgSO₄) and evaporate the solvent *in vacuo* to give the title compound.

### Example 28

### N,N-di[4-Dimethylaminophenyl]-4-methyl-1-piperazineacetamide dioxalate

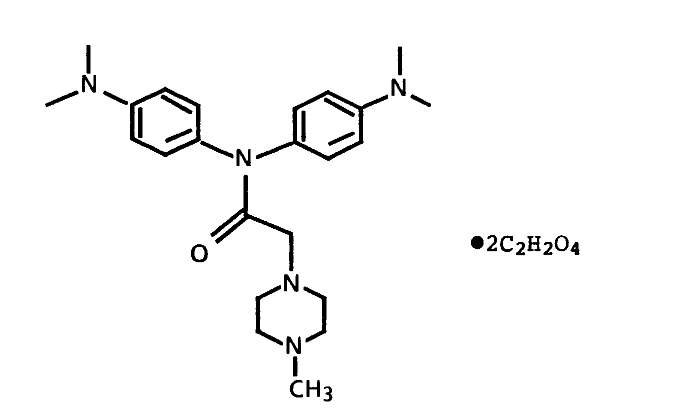

Prepare as described above in Example 2 for N,N-diphenyl-4-methyl-1-piperazineacetamide dioxalate but using 4,4'-dimethylaminodiphenylamine instead of diphenylamine.

### Example 29

### N-3-Chlorophenyl-N-phenyl-4-methyl-1-piperazineaceamide dioxalate

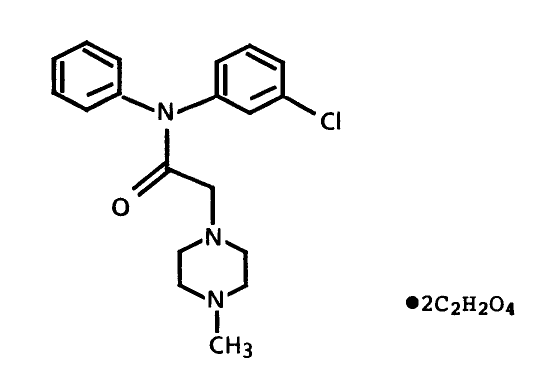

Prepare as described above in Example 2 for N,N-diphenyl-4-methyl-1-piperazineacetamide dioxalate but using 3-chloro-N,N-diphenylamine instead of diphenylamine.

### Example 30

### N-2-Methoxycarbonylphenyl-N-phenyl-4-methyl-1-piperazineacetamide dioxalate

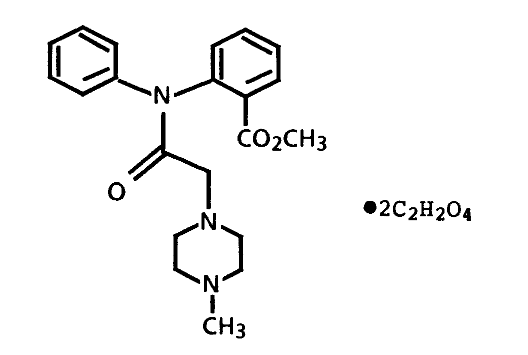

Prepare as described above in Example 13 for N-(3-methoxycarbonylmethylphenyl-N-phenyl-4-methyl-1-piperazineacetamide dioxalate but using N-(2-methoxycarbonylphenyl)-N-phenylamine instead of N-(3-methoxycarbonylmethylphenyl)-N-phenylamine.

### Example 31

### N-2-Carboxyphenyl-N-phenyl-4-methyl-1-piperazineacetamide dihydrochloride

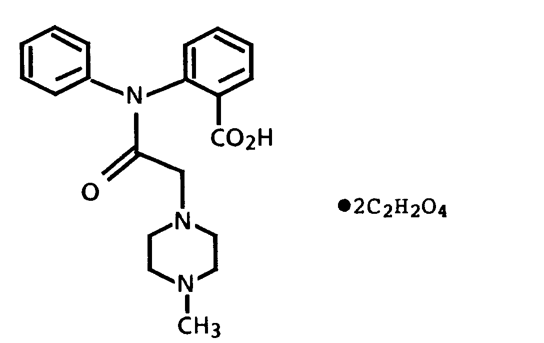

Prepare as described above in Example 14 for N-(3-carboxymethylphenyl-N-phenyl-4-methyl-1-piperazineacetamide dioxalate but using N-(2-methoxycarbonylmethylphenyl-N-phenyl-4-methyl-1-piperazineacetamide instead of N-(3-methoxycarbonylmethylphenyl-N-phenyl-4-methyl-1-piperazineacetamide.

### Example 32

### N,N-di[4-methoxyphenyl]-4-methyl-1-piperazineacetamide dioxalate

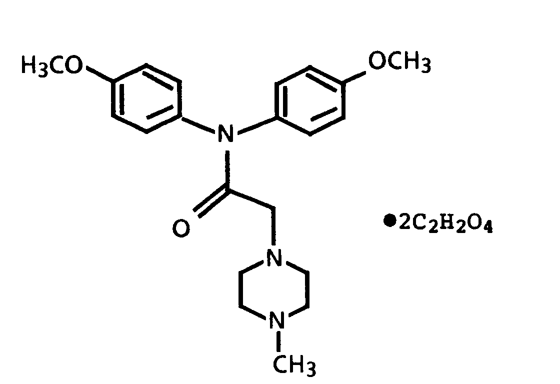

Prepare as described above in Example 2 for N,N-diphenyl-4-methyl-1-piperazineacetamide dioxalate but using 4,4'-dimethoxy-N,N-diphenylamine instead of diphenylamine.

### Example 33

### N-3-Trifluoromethylphenyl-N-phenyl-4-methyl-1-piperazineacetamide dioxalate

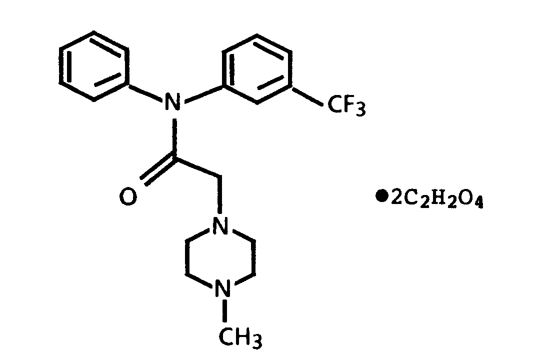

Prepare as described above in Example 2 for N,N-diphenyl-4-methyl-1-piperasineacetamide dioxalate but using 3-trifluromethyl-N,N-diphenylamine instead of diphenylamine.

### Example 34

### N-4-Cyclohexylaminophenyl-N-phenyl-4-methyl-1-piperazineaceamide dioxalate

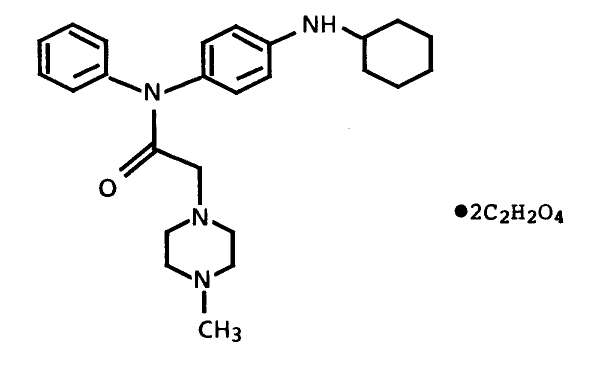

Prepare as described above in Example 18 for N-4-methylaminophenyl-N-phenyl-4-methyl-1-piperazineacetamide dioxalate but using 4-cyclohexylamino-N,N-diphenylamine instead of N-[4-methylaminophenyl]-N-phenylamine.

### Example 35

### N-4-Sulfophenyl-N-phenyl-4-methyl-1-piperazineacetamide dihydrochloride

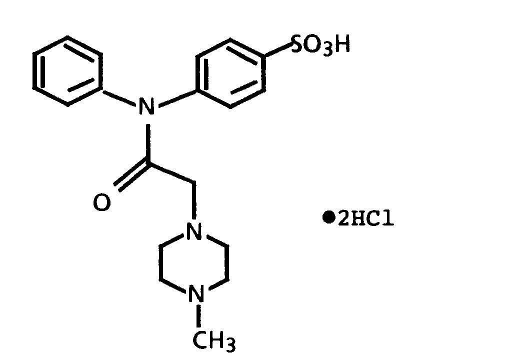

### Step a: N-4-Sulfophenyl-N-phenylchloroacetamide

Dissolve diphenylamine-4-sulfonic acid (10mmol) in acetonitrile (100mL) and add pyridine (40mmol). Cool in an ice bath and slowly add chloroacetyl chloride (3.1mL, 40mmol). Remove the cooling bath and stir at room temperature overnight under a nitrogen atmosphere. Add saturated aqueous sodium hydrogen carbonate and stir vigorously for 3 hours. Evaporate the solvent *in vacuo*, acidify the residue with 1N hydrochloric acid and add ethyl acetate. Separate the organic phase, dry (MgSO₄) and evaporate the solvent *in vacuo* to give the title compound.

### Step b: N-4-Sulfophenyl-N-phenyl-4-methyl-1-piperazineacetamide dihydrochloride

Dissolve N-4-sulfophenyl-N-phenylchloroacetamide (5mmol) in acetonitrile (75mL), add N-methyl piperazine (1.2g, 12mmol) and reflux overnight under a nitrogen atmosphere. Evaporate the solvent *in vacuo*, take the residue up in water and place on a column of ion exchange resin. Elute with water followed by 1N hydrochloric acid. Evaporate the water from the acidic phase and recrystallize (ethanol) to give the title compound.

### Example 36

### N-3-Methoxyphenyl-N-phenyl-4-methyl-1-piperazineacetamide dioxalate

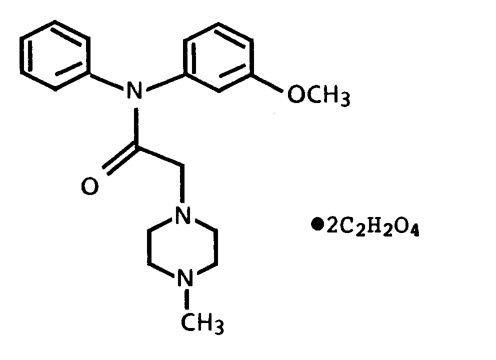

Prepare as described above in Example 2 for N,N-diphenyl-4-methyl-1-piperazineacetamide dioxalate but using 3-methoxy-N,N-diphenylamine instead of diphenylamine.

### Example 37

### N-2-Fluorophenyl-N-(2-pyrinyl)-4-methyl-1-piperazineacetamide dioxalate

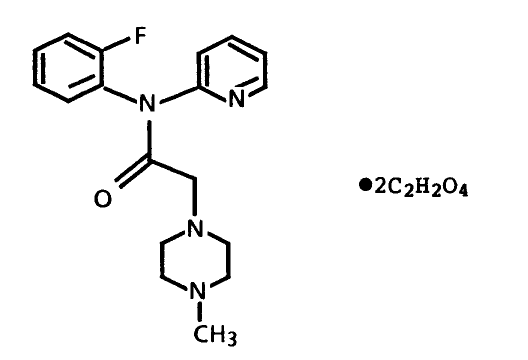

Prepare as described above in Example 2 for N,N-diphenyl-4-methyl-1-piperazineacetamide dioxalate but using N-2-fluorophenyl-N-(2-pyridyl)amine instead of diphenylamine.

### Example 38

### N,N-di-[4-methylphenyl]-4-methyl-1-piperazineacetamide dioxalate

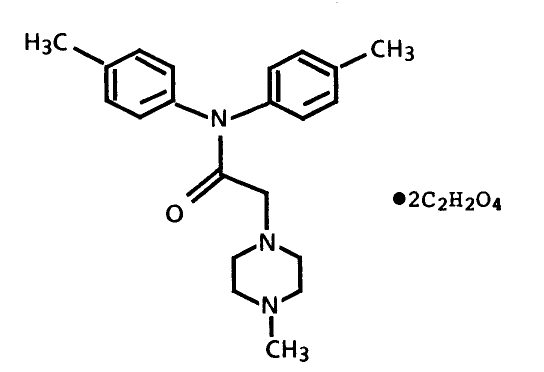

Prepare as described above in Example 2 for N,N-diphenyl-4-methyl-1-piperazineacetamide dioxalate but using 4,4'-dimethyl-N,N-diphenylamine instead of diphenylamine.

### Example 39

### N-4-Propionamidophenyl-N-phenyl-4-methyl-1-piperazineacetamide dioxalate

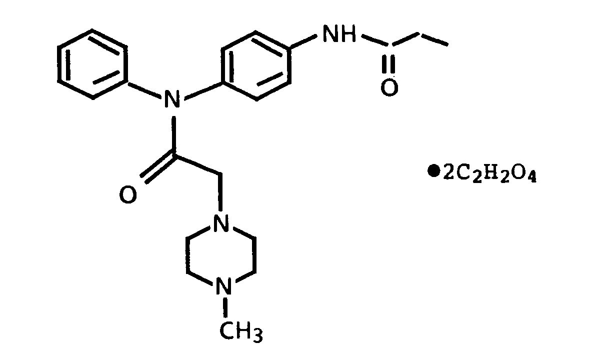

Prepare as described above in Example 2 for N,N-diphenyl-4-methyl-1-piperazineacetamide dioxalate but using 4-propionamido-N,N-diphenylamine instead of diphenylamine.

### Example 40

### N-4-Maleamidophenyl-N-phenyl-4-methyl-1-piperazineacetamide diformate

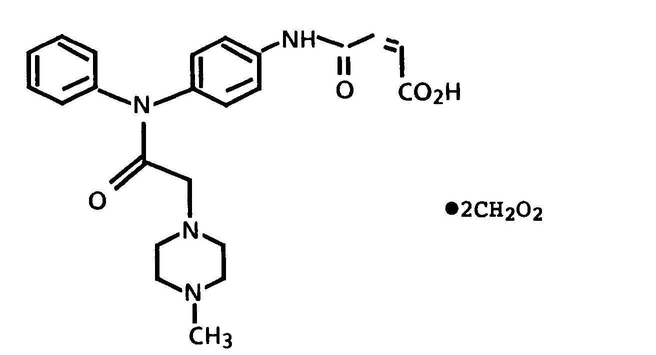

Prepare as described above in Example 5 for 4-acetamido-N,N-diphenyl-4-methyl-1-piperazineacetamide dioxalate but using maleic anhydride instead of acetyl chloride; the title compound is purified by reversed phase HPLC (H₂O, CH₃CN, HCOOH) followed by recrystallization.

### Example 41

### N-4-Glutaramidophenyl-N-phenyl-4-methyl-1-piperazineacetamide diformate

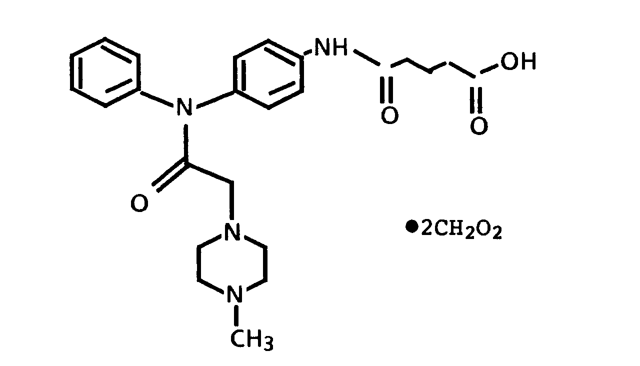

Prepare as described above in Example 40 for N-4-maleamidophenyl-N-phenyl-4-methyl-1-piperazineacetamide diformate but using glutaric anhydride instead of maleic anhydride.

### Example 42

### N-4-Isopropylaminophenyl-N-phenyl-4-methyl-1-piperazineacetamide dioxalate

Prepare as described above in Example 18 for N-4-methylaminophenyl-N-phenyl-4-methyl-1-piperazineacetamide dioxalate but using 4-isopropylamino-N,N-diphenylamine instead of N-[4-methylaminophenyl]-N-phenylamine.

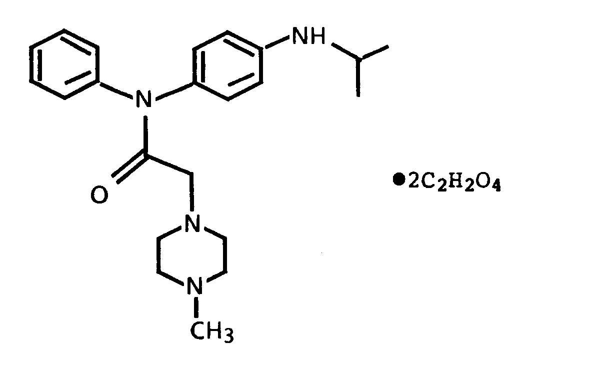

### Example 43

### N-4-(p-Toluenesulfamido)phenyl-N-diphenyl-4-methyl-1-piperazineacetamide dioxalate

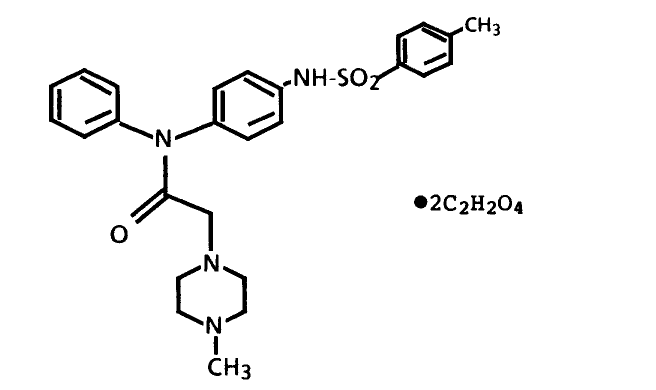

Prepare as described above in Example 2 for N,N-diphenyl-4-methyl-1-piperazineacetamide dioxalate but using 4-(p-toluenesulfamido)-N,N-diphenylamine instead of diphenylamine.

Alternatively, prepare as described above in Example 5 for N-4-acetamidophenyl-N-phenyl-4-methyl-1-piperazineacetamide dioxalate but using p-toluenesulfonylchloride instead of acetylchloride.

### Example 44

### N-4-(2-Tetrazoyl)phenyl-N-phenyl-4-methyl-1-piperazineacetamide dioxalate

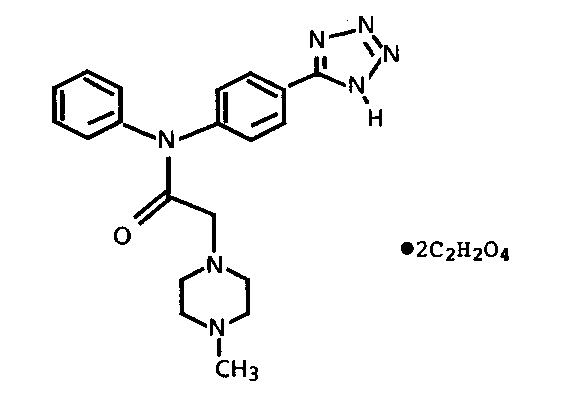

Mix N-4-cyanophenyl-N-phenyl-4-methyl-1-piperazineacetamide (59.4mmol), sodium azide (3.9g, 59.4mmol), ammonium chloride (3.2g, 59.4mmol) and dimethylformamide (30mL). Heat at 115-120°C for 4 hours. Cool and add another 3 equivalents of both sodium aside and ammonium chloride. Heat an additional 18 hours at 115-120°C. Pour into water (500mL) and extract with ethyl acetate. Separate the organic phase and wash repeatedly with water then a brine solution. Dry (MgSO₄) and evaporate the solvent *in vacuo*. Purify by chromatography to give N-4-(2-tetrazoyl)phenyl-N-phenyl-4-methyl-1-piperazineacetamide.

Dissolve N-4-(2-tetrazoyl)phenyl-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize to give the title compound.

### Example 45

### N-3-(t-Butoxycarbonyl)methoxyphenyl-N-phenyl-4-methyl-1-piperazineacetamide dioxalate

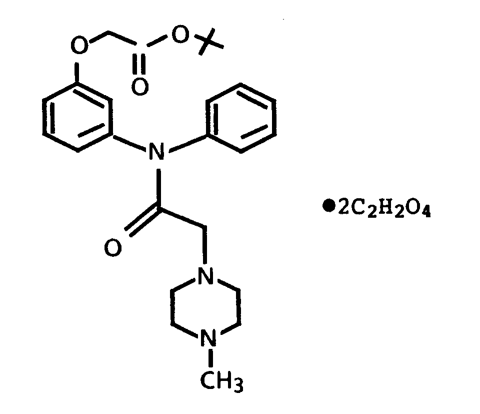

### Step a: N-[3-(t-butyloxycarbonylmethoxy)phenyl]-N-phenylchloroacetamide

Mix 3-hydroxy-diphenylamine (3.7g, 20mmol), potassium carbonate (8.3g), α-bromo-tert-butylacetate (3.22mL, 22mmol) and acetone (40mL). Reflux for 24 hours, add water and ethyl ether and separate the organic phase. Dry (MgSO₄) and evaporate the solvent *in vacuo* to give 3-(t-butoxycarbonylmethoxy)-diphenylamine.

Dissolve 3-(t-butoxycarbonylmethoxy)-diphenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate.

Separate the organic phase, dry (MgSO₄) and evaporate the solvent *in vacuo* to give the title compound.

### Step b: N-3-(t-Butoxycarbonyl)methoxyphenyl-N-phenyl-4-methyl-1-piperazineacetamide dioxalate

Dissolve N-[3-(t-butoxycarbonylmethoxy)phenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-3-(t-butoxycarbonyl)methoxyphenyl-N-phenyl-4-methyl-1-piperazineacetamide.

Dissolve N-3-(t-butoxycarbonyl)methoxyphenyl-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize to give the title compound.

### Example 46

### N-4-Carboxymethoxyphenyl-N-phenyl-4-methyl-1-piperazineacetamide dioxalate

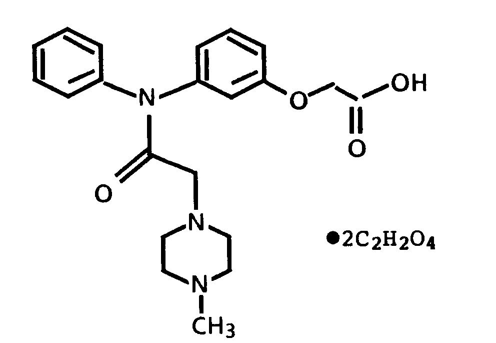

Mix N-3-(t-butoxycarbonyl)methoxyphenyl-N-phenyl-4-methyl-1-piperazineacetamide (500mg) and oxalic acid (200mg) in water and heat for 2 hours. Evaporate the water *in vacuo* and recrystallize the residue (isopropanol) to give the title compound.

### Example 47

### N-3-(2-Diethylamino)ethoxyphenyl-N-phenyl-4-methyl-1-piperazineacetamide trioxalate

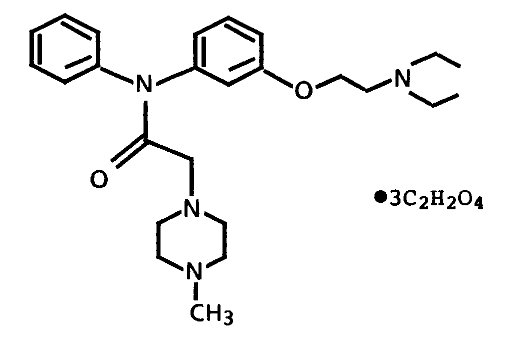

### Step a: N-[3-(2-Diethylaminoethoxy)phenyl]-N-phenylchloroacetamide

Suspend lithium aluminum hydride (2.3g, 60mmol) in tetrahydrofuran (150mL) and add, by dropwise addition, a solution of 3-(t-butoxycarbonylmethoxy)-diphenylamine (15g, 50mmol) in tetrahydrofuran (50mL). Maintain reflux for 1 hour, cool and slowly add a mixture of Na₂SO₄·10H₂O (15g) and filter aid (15g). Stir the mixture overnight, filter through filter aid and evaporate the solvent *in vacuo* to give N-[3-(2-hydroxyethoxy)phenyl]-N-phenylamine.

Dissolve carbon tetrabromide (869mg, 2.62mmol) in methylene chloride (7mL) and add a solution of triphenylphosphine (687mg, 2.62mmol) in methylene chloride (6mL). Stir for 15 minutes and add a solution of N-[3-(2-hydroxyethoxy)phenyl]-N-phenylamine (500mg, 2.18mmol) in methylene chloride (7mL). Stir for 48 hours and add additional triphenylphosphine (2.62mmol) and carbon tetrabromide (2.62mmol). Stir overnight and purify by chromatography (20% ethyl acetate/hexane) to give N-[3-(2-bromoethoxy)phenyl]-N-phenylamine.

Dissolve N-[3-(2-bromoethoxy)phenyl]-N-phenylamine in diethylamine and leave at room temperature for 48 hours. Evaporate the diethylamine *in vacuo*, extract into ethyl acetate and evaporate the solvent *in vacuo* to give N-[3-(2-diethylaminoethoxy)phenyl]-N-phenylamine.

Dissolve N-[3-(2-diethylaminoethoxy)phenyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography to give the title compound.

### Step b: N-3-(2-Diethylamino)ethoxyphenyl-N-phenyl-4-methyl-1-piperazineacetamide trioxalate

Dissolve N-[3-(2-diethylaminoethoxy)phenyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-3-(2-diethylamino)ethoxyphenyl-N-phenyl-4-methyl-1-piperazineacetamide.

Dissolve N-3-(2-diethylamino)ethoxyphenyl-N-phenyl-4-methyl-1-piperazineacetamide (1.5mmol) in tetrahydrofuran (50mL) and add a solution of oxalic acid dihydrate (600mg, 4.5mmol) in tetrahydrofuran (15mL). Filter the precipitate and recrystallize to give the title compound.

### Example 48

### N-3-[2-((4-Methyl-1-piperazinomethyl)carboxy)ethoxy]phenyl-N-phenyl-4-methyl-1-piperazineacetamide tetraoxalate

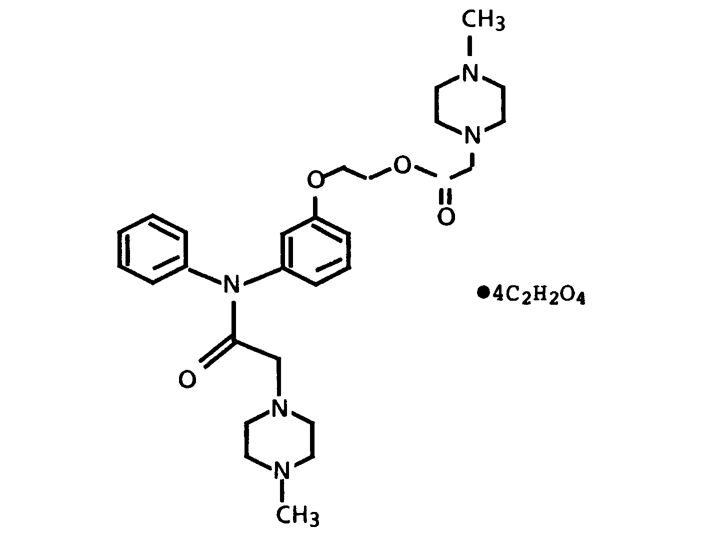

### Step a: N-[3-(2-((4-Methyl-1-piperazinomethyl)carboxy)ethoxy])phenyl]-N-phenylchloroacetamide

Dissolve N-[3-(2-hydroxyethoxy)phenyl]-N-phenylamine (38mmol) in acetonitrile (450mL) and add pyridine (9.2mL, 114mmol). Cool in an ice-bath and slowly add chloroacetyl chloride (9mL, 114mmol). Remove the cooling bath and stir at room temperature overnight under a nitrogen atmosphere.

Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography to give the title compound.

### Step b: N-3-[2-((4-Methyl-1-piperazinomethyl)carboxy)ethoxy]phenyl-N-phenyl-4-methyl-1-piperazineacetamide tetraoxalate

Dissolve N-[3-(2-((4-methyl-1-piperazinomethyl)carboxy)ethoxy])phenyl]-N-phenylchloroacetamide (30mmol) in acetonitrile (150mL), add N-methylpiperazine (24g, 240mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solven*t in vacuo* to give N-3-[2-((4-methyl-1-piperazinomethyl)carboxy)ethoxy]phenyl-N-phenyl-4-methyl-1-piperazineacetamide.

Dissolve N-3-[2-((4-methyl-1-piperazinomethyl)carboxy)ethoxy]phenyl-N-phenyl-4-methyl-1-piperazineacetamide (1.5mmol) in tetrahydrofuran (50mL) and add a solution of oxalic acid dihydrate (800mg, 6mmol) in tetrahydrofuran (15mL). Filter the precipitate and recrystallize to give the title compound.

### Example 49

### N-3-(2-Hydroxyethoxy)-N,N-diphenyl-4-methyl-1-piperazineacetamide dioxalate

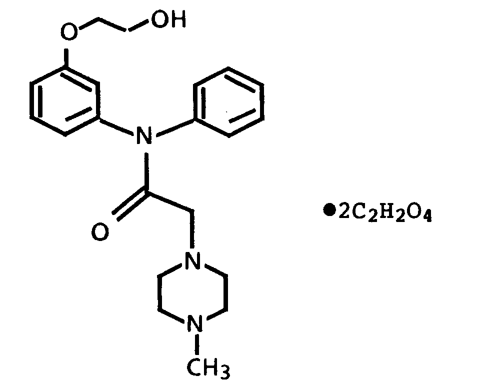

Mix N-3-[2-((4-methyl-1-piperazinomethyl)carboxy)ethoxy]phenyl-N-phenyl-4-methyl-1-piperazineacetamide (44mmol), potassium carbonate (20g) and a mixture of 1:4 water/methanol (300mL). Stir at room temperature for 24 hours, evaporate the solvent *in vacuo* and partition between water and ethyl ether. Separate the organic phase, dry (MgSO₄) and evaporate the solvent *in vacuo* to give N-3-(2-hydroxyethoxy)phenyl-N-phenyl-4-methyl-1-piperazineacetamide.

Dissolve N-3-(2-hydroxyethoxy)phenyl-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize to give the title compound.

### Example 50

### N-4-Methylglutaramidophenyl-N-phenyl-4-methyl-1-piperazineacetamide dioxalate

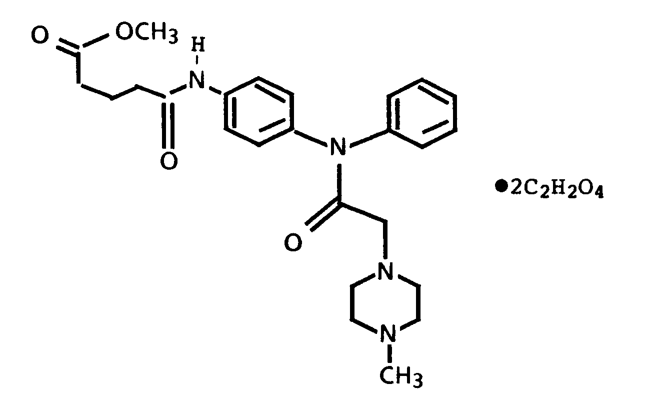

Mix N-4-aminophenyl-N-phenyl-4-methyl-1-piperazineacetamide (200mg, 0.6mmol), triethylamine (0.17mL), methyl glutaryl chloride (0.6mmol), dimethylaminopyridine (5mg) and acetonitrile (10mL). Stir at room temperature for 24 hours, evaporate the solvent in vacuo and partition between saturated aqueous sodium hydrogen carbonate and ethyl acetate. Separate the organic phase, dry (MgSO₄) and evaporate the solvent *in vacuo* to give N-4-methylglutaramido-phenyl-N-phenyl-4-methyl-1-piperazineacetamide.

Dissolve N-4-methylglutaramidophenyl-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize (isopropanol/water) to give the title compound.

### Example 51

### N-4-Methylsuccinamidophenyl-N-phenyl-4-methyl-1-piperazineacetamide dioxalate

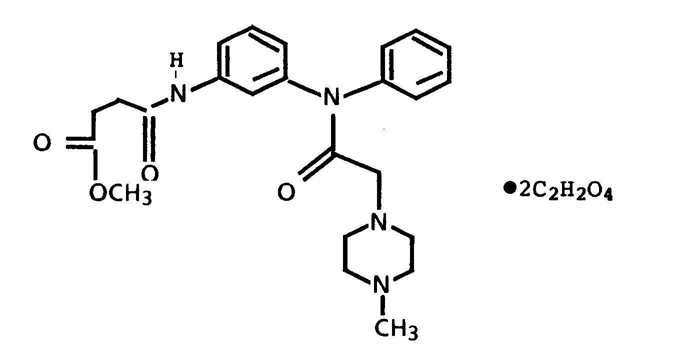

Mix N-4-aminophenyl-N-phenyl-4-methyl-1-piperazineacetamide (200mg, 0.6mmol), triethylamine (0.17mL), methyl succinyl chloride (0.6mmol), dimethylaminopyridine (5mg) and acetonitrile (10mL). Stir at room temperature for 24 hours, evaporate the solvent *in vacuo* and partition between saturated aqueous sodium hydrogen carbonate and ethyl acetate. Separate the organic phase, dry (MgSO₄) and evaporate the solvent *in vacuo* to give N-4-methylsuccinamidophenyl-N-phenyl-4-methyl-1-piperazineacetamide.

Dissolve N-4-methylsuccinamidophenyl-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize (isopropanol/water) to give the title compound.

### Example 52

### N-4-Pyrrolidinophenyl-N-phenyl-4-methyl-1-piperazineacetamide trioxalate

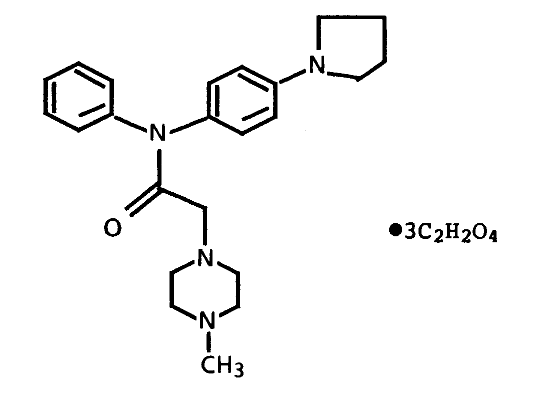

### Step a: N-4-Pyrrolidinophenyl-N-phenylchloroacetamide

Mix 4-aminodiphenylamine (500mg, 2.7mmol), 1,4-dibromobutane (0.32mL, 2.7mmol), potassium carbonate (2g) and dimethylformamide. Heat at 80°C overnight, cool to room temperature and add water (20mL). Separate the aqueous phase and extract with a 1:1 mixture of ethyl ether/cyclohexane. Combine the organic phases, dry (MgSO₄) and evaporate the solvent *in vacuo*. Purify by chromatographjy (20:80 ethyl acetate/cyclohexane) to give 4-pyrrolidino-diphenylamine (400mg).

Dissolve 4-pyrrolidino-diphenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo* to give the title compound.

### Step b: N-4-Pyrrolidinophenyl-N-phenyl-4-methyl-1-piperazineacetamide trioxalate

Dissolve 4-pyrrolidino-N,N-Diphenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-4-pyrrolidinophenyl-N-phenyl-4-methyl-1-piperazineacetamide.

Dissolve N-4-pyrrolidinophenyl-N-phenyl-4-methyl-1-piperazineacetamide (1.5mmol) in tetrahydrofuran (50mL) and add a solution of oxalic acid dihydrate (600mg, 4.5mmol) in tetrahydrofuran (15mL). Filter the precipitate and recrystallize (ethanol/water) to give the title compound.

### Example 53

### N-4-Dimethylaminophenyl-N-benzyl-4-methyl-1-piperazineacetamide dioxalate

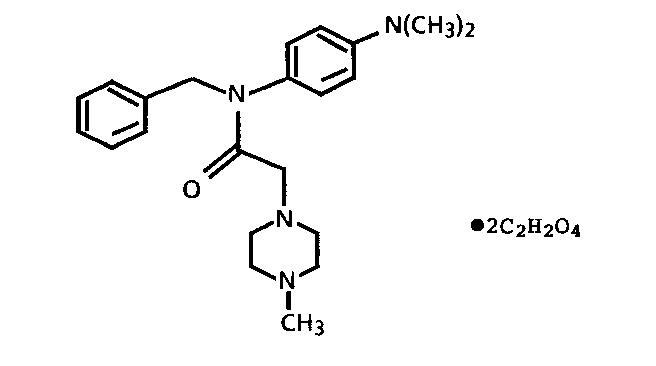

### Step a: N-4-Dimethylaminophenyl-N-benzylchloroacetamide

Mix N,N-dimethyl-4-phenylenediamine dihydrochloride (2g), potassium carbonate (10g), benzyl bromide (1.7g) and acetone (50mL). Reflux for 24 hours, add water and ethyl ether and separate the organic phase, dry (MgSO₄) and evaporate the solvent *in vacuo*. Purify by chromatography (2:8 ethyl acetate/cyclohexane followed by 5:5 ethyl acetate/cyclohexane) to give N-4-dimethylaminophenyl-N-benzylamine.

Dissolve N-4-dimethylaminophenyl-N-benzylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography to give the title compound.

### Step b: N-4-Dimethylaminophenyl-N-benzyl-4-methyl-1-piperazineacetamide dioxalate

Dissolve N-4-dimethylaminophenyl-N-benzylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-4-dimethylaminophenyl-N-benzyl-4-methyl-1-piperazineacetamide.

Dissolve N-4-dimethylaminophenyl-N-benzyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize to give the title compound.

### Example 54

### N-4-(2-1H-Imidazoyl)phenyl-N-phenyl-4-methyl-1-piperazineacetamide dioxalate

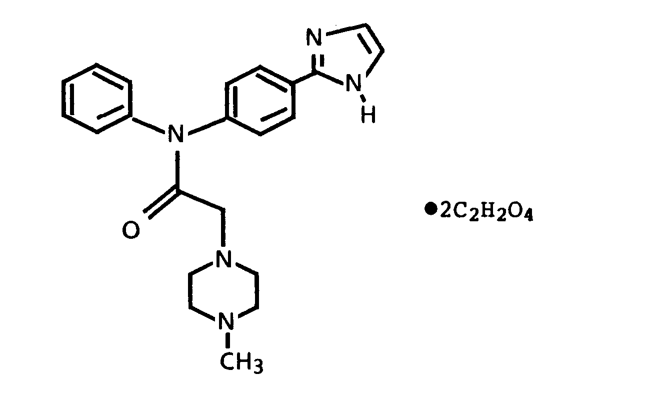

Mix N-4-cyanophenyl-N-phenyl-4-methyl-1-piperazineacetamide (113mmol), sulfur (1.9g, 0.06mol), 2-methoxyethanol (100mL) and ethylene diamine (8.4g, 0.14mol) and heat at 130°C for 5 hours. Dilute with 1N hydrochloric acid and wash with ethyl acetate. Neutralize the aqueous phase with potassium carbonate and extract into ethyl acetate. Dry (MgSO₄) and evaporate the solvent *in vacuo* to give N-4-[2-(4,5-dihydro)-1H-imidazole]phenyl-N-phenyl-4-methyl-1-piperazineacetamide.

Heat a mixture of MnO₂ (7.5g, 85mmol) and benzene (50mL) for 2 hours, collecting any water present in a Dean-Stark trap. Cool, decant the benzene and add a solution of N-4-[2-(4,5-dihydro)-1H-imidazole]phenyl-N-phenyl-4-methyl-1-piperazineacetamide (7.2mmol) in methylene chloride (30mL). Stir for 6 days, filterd and extract with methylene chloride in a Soxhlet apparatus for 24 hours. Combine the organic extracts and evaporate the solvent *in vacuo* to give N-4-(2-1H-imidazole)phenyl-N-phenyl-4-methyl-1-piperazineacetamide.

Dissolve N-4-(2-1H-imidazole)phenyl-N-phenyl-4-methyl-1-piperazineacetamide (1.5mmol) in tetrahydrofuran (50mL) and add a solution of oxalic acid dihydrate (400mg, 3mmol) in tetrahydrofuran (20mL). Filter the precipitate and recrystallize to give the title compound.

### Example 55

### N-6-(t-Butoxycarbamoyl-3-pyridyl)-N-phenyl-4-methyl-1-piperazineacetamide dioxalate

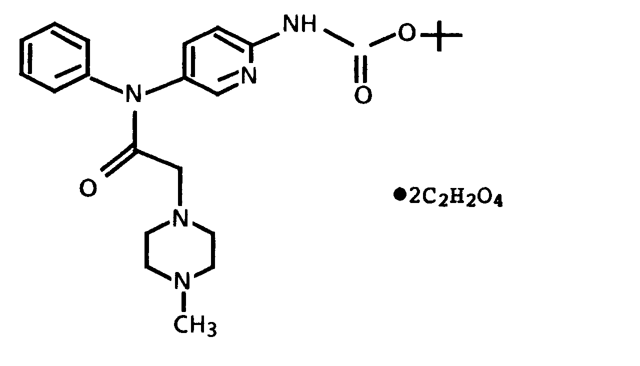

### Step a: N-[6-(t-Butoxycarbamoyl)-3-pyridyl]-N-phenylchloroacetamide

Dissolve 2-amino-5-bromopyridine (54mmol) in chloroform (100mL), add triethylamine (11mL, 81mmol) and cool in an ice-bath. Slowly add a solution of di-tert-butyldicarbonate (12g, 54mmol) in chloroform (50mL), remove the ice-bath and stir at room temperature overnight under a nitrogen atmosphere. Evaporate the solvent *in vacuo* and purify by chromatography to give 2-(t-Butoxycarbamoyl)-5-bromopyridine.

Suspend sodium amide (208mmol) in aniline (20ml) and add, by dropwise addition, t-butanol at room temperature. Heat at 45-50°C for 2 hours. Add a solution of 2-(t-Butoxycarbamoyl)-5-bromopyridine (54mmol) in aniline (20mL) and stir at 40°C for 5 hours. Pour onto cracked ice, extract into ethyl ether and dry (MgSO₄). Evaporate the solvent *in vacuo* to give N-6-[t-Butoxycarbamoyl-3-pyridyl]-N-phenylamine.

Dissolve N-6-[t-Butoxycarbamoyl-3-pyridyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography to give the title compound.

### Step b: N-6-[t-Butoxycarbamoyl-3-pyridyl]-N-phenyl-4-methyl-1-piperazineacetamide dioxalate

Dissolve N-[6-(t-Butoxycarbamoyl)-3-pyridyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and parition between saturated aqueous sodium hydrogen carbonate and ethyl acetate. Separate the organic phase, wash with brine, dry (MgSO₄) and evaporate the solvent *in vacuo* to give N-6-[(t-Butoxycarbamoyl)3-pyridyl]-N-phenyl-4-methyl-1-piperazineacetamide.

Dissolve N-6-[t-Butoxycarbamoyl-3-pyridyl]-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize to give the title compound.

### Example 56

### N-[6-Amino-3-pyridyl-N-phenyl-4-methyl-1-piperazineacetamide trioxalate

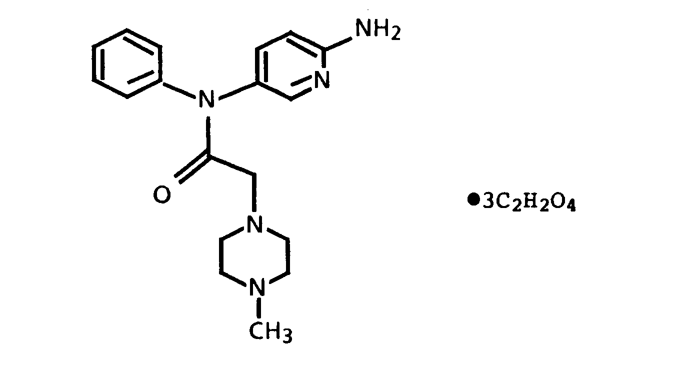

Dissolve N-6-[t-Butoxycarbamoyl-3-pyridyl]-N-phenyl-4-methyl-1-piperazineacetamide (14mmol) in a 40:60 mixture of methylene chloride/trifluoroacetic acid (200mL), cool in an ice-bath and stir for 1 hour. Evaporate the solvent *in vacuo* and partition between saturated aqueous sodium hydrogen carbonate and ethyl acetate. Separate the organic phase, dry (MgSO₄) and evaporate the solvent *in vacuo* to give N-6-[amino-3-pyridyl]-N-phenyl-4-methyl-1-piperazineacetamide.

Dissolve N-[6-amino-3-pyridyl]-N-phenyl-4-methyl-1-piperazineacetamide (1.5mmol) in tetrahydrofuran (50mL) and add a solution of oxalic acid dihydrate (600mg, 4.5mmol) in tetrahydrofuran (20mL). Filter the precipitate and recrystallize to give the title compound.

### Example 57

### N-[6-t-Butoxycarbamoyl-2-pyridyl]-N-phenyl-4-methyl-1-piperazineacetamide dioxalate

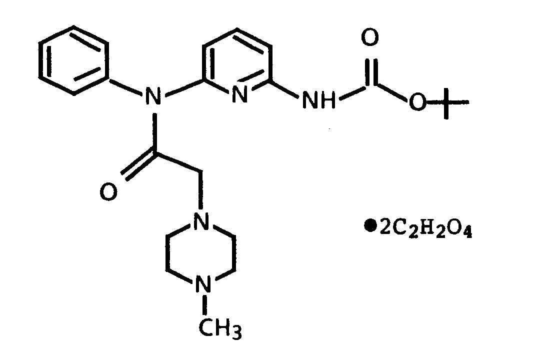

### Step a: N-[6-(t-Butoxycarbamoyl)-2-pyridyl]-N-phenylchloroacetamide

Mix 2,6-diaminopyridine (5.5g, 50mmol), bromobenzene (10mmol, 1.05mL), potassium carbonate (1.5g) and CuI (0.2g). Heat at 180°C for 24 hours under a nitrogen atmosphere. Purify by chromatography (3:1 ethyl acetate/cyclohexane) to give N-[6-amino-2-pyridyl]-N-phenylamine.

Dissolve N-[6-amino-2-pyridyl]-N-phenylamine (54mmol) in chloroform (100mL), add triethylamine (11mL, 81mmol) and cool in an ice-bath. Slowly add a solution of di-tert-butyldicarbonate (12g, 54mmol) in chloroform (50mL), remove the ice-bath and stir at room temperature overnight under a nitrogen atmosphere. Evaporate the solvent *in vacuo* and purify by chromatography to give N-[6-(t-Butoxycarbamoyl)-2-pyridyl]-N-phenylamine.

Dissolve N-[6-(t-Butoxycarbamoyl)-2-pyridyl]-N-phenylamine (38mmol) in acetonitrile (450mL) and add pyridine (4.6mL, 57mmol). Cool in an ice-bath and slowly add chloroacetyl chloride (4.5mL, 57mmol). Remove the cooling bath and stir at room temperature overnight under a nitrogen atmosphere. Evaporate the solvent *in vacuo* and partition between saturated aqueous sodium hydrogen carbonate and ethyl acetate. Separate the organic phase, dry (MgSO₄) and evaporate the solvent *in vacuo*. Purify by chromatography to give the title compound.

### Step b: N-6-[t-Butoxycarbamoyl-2-pyridyl]-N-phenyl-4-methyl-1-piperazineacetamide dioxalate

Dissolve N-[6-(t-Butoxycarbamoyl)-2-pyridyl]-N-phenylchloroacetamide (30mmol) in acetonitrile (150mL), add N-methylpiperazine (12g, 120mmol) and reflux overnight under a nitrogen atmosphere. Evaporate the solvent *in vacuo* and partition between saturated aqueous sodium hydrogen carbonate and ethyl acetate. Separate the organic phase, wash with brine, dry (MgSO₄) and evaporate the solvent *in vacuo* to give N-6-[t-Butoxycarbamoyl-2-pyridyl]-N-phenyl-4-methyl-1-piperazineacetamide.

Dissolve N-6-[t-Butoxycarbamoyl-2-pyridyl]-N-phenyl-4-methyl-1-piperazineacetamide (1.5mmol) in tetrahydrofuran (50mL) and add a solution of oxalic acid dihydrate (400mg, 3mmol) in tetrahydrofuran (15mL). Filter the precipitate and recrystallize to give the title compound.

### Example 58

### N-[6-Amino-2-pyridyl]-N-phenyl-4-methyl-1-piperazineacetamide trioxalate

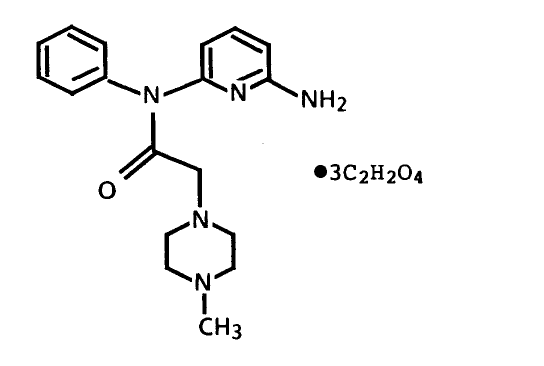

Dissolve N-6-[t-Butoxycarbamoyl-2-pyridyl]-N-phenyl-4-methyl-1-piperazineacetamide (14mmol) in a 40:60 mixture of methylene chloride/trifluoroacetic acid (200mL), cool in an ice-bath and stir for 1 hour. Evaporate the solvent *in vacuo* and partition between saturated aqueous sodium hydrogen carbonate and ethyl acetate. Separate the organic phase, dry (MgSO₄) and evaporate the solvent *in vacuo* to give N-6-[amino-2-pyridyl]-N-phenyl-4-methyl-1-piperazineacetamide.

Dissolve N-[6-amino-2-pyridyl]-N-phenyl-4-methyl-1-piperazineacetamide (1.5mmol) in tetrahydrofuran (50mL) and add a solution of oxalic acid dihydrate (600mg, 4.5mmol) in tetrahydrofuran (20mL). Filter the precipitate and recrystallize to give the title compound.

### Example 59

### N-3-Dimethylaminosulfonylphenyl-N-phenyl-4-methyl-1-piperazineacetamide dioxalate

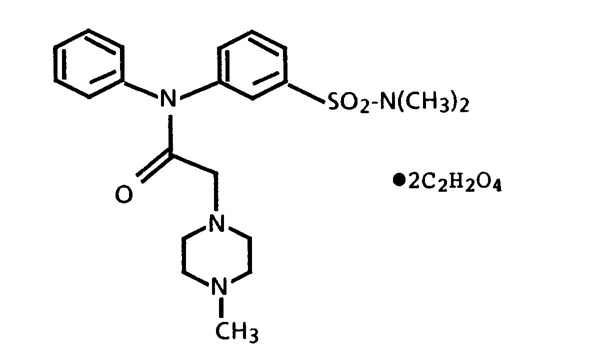

Dissolve N,N-diphenylamino-3-sulfonic acid-piperazineacetamide (40mmol) in methylene chloride (200mL). Cool to 0°C, place under a nitrogen atmosphere and add, by dropwise addition, thionyl chloride (5.28g, 44mmol). Stir at room temperature for several hours and evaporate the solvent *in vacuo* to give 3-chlorosulfonyl-N,N-diphenylamine.

Dissolve dimethylamine hydrochloride (0.67mmol) in saturated sodium hydrogen carbonate (3mL). Add a solution of 3-chlorosulfonyl-N,N-diphenylamine(0.34mmol) in acetone (3mL) and stir at room temperature under an argon atmosphere for 30 minutes. Adjust to pH 6 with saturated sodium hydrogen carbonate and extract into ethyl ether. Dry (MgSO₄) and evaporate the solvent *in vacuo* to give 3-dimethylaminosulfonyl-N,N-diphenylamine.

N-3-dimethylaminosulfonylphenyl-N-phenyl-4-methyl-1-piperaineacetamide dioxalate is prepared as described above in example 2 for N,N-diphenyl-4-methyl-1-piperazine acetamide dioxalate but using 3-dimethylammosulfonyl-N,N-diphenylamine instead of diphenylamine.

The following compounds may be prepared by procedures analogous to those described above for Examples 1-59:
N-2-Aminophenyl-N-phenyl-4-methyl-1-piperazineacetamide;
N-4-Methoxyphenyl-N-benzyl-4-methyl-1-piperazineacetamide;
N-2-Ethoxyphenyl-N-benzyl-4-methyl-1-piperazineacetamide;
N-4-Hydroxyphenyl-N-benzyl-4-methyl-1-piperazineacetamide;
N-2-Dimethylaminomethylphenyl-N-benzyl-4-methyl-1-piperazineacetamide;
N-2-hydroxyphenyl-N-benzyl-4-methyl-1-piperazineacetamide;
N-2-Ethoxycarbonylphenyl-N-3-aminobenzyl-4-methyl-1-piperazineacetamide;
N-4-Ethoxyphenyl-N-4-dimethylamino-N-benzyl-4-methyl-1-piperazineacetamide;
N-4-Ethoxycarbonylphenyl-N-4-dimethylaminobenzyl-4-methyl-1-piperazineacetamide;
N-4-Chlorophenyl-N-4-hydroxybenzyl-4-methyl-1-piperazineacetamide;
N-4-Methoxyphenyl-N-4-hydroxybenzyl-4-methyl-1-piperazineacetamide;
N-4-Ethoxyphenyl-N-4-hydroxybenzyl-4-methyl-1-piperazineacetamide;
N-4-Ethoxycarbonylphenyl-N-4-hydroxybenzyl-4-methyl-1-piperazineacetamide;
N-4-Methylphenyl-N-4-hydroxybenzyl-4-methyl-1-piperazineacetamide;
N-4-Ethylphenyl-N-4-hydroxybenzyl-4-methyl-1-piperazineacetamide;
N-4-Fluorophenyl-N-4-hydroxybenzyl-4-methyl-1-piperazineacetamide;
N-4-Ethoxyphenyl-N-4-chloro-N-benzyl-4-methyl-1-piperazineacetamide;
N-4-Bromophenyl-N-4-hydroxybenzyl-4-methyl-1-piperazineacetamide;
N-4-Ethoxyphenyl-N-4-methoxybenzyl-4-methyl-1-piperazineacetamide;
N-4-Methylphenyl-N-4-methoxybenzyl-4-methyl-1-piperazineacetamide;
N-4-methylphenyl-N-benzyl-4-methyl-1-piperazineacetamide;
N-4-Methoxyphenyl-N-4-methoxybenzyl-4-methyl-1-piperazineacetamide;
N-4-methoxyphenyl-N-benzyl-4-methyl-1-piperazineacetamide;
N-4-Aminophenyl-N-4-(p-toluenesulfamide)benzyl-4-methyl-1-piperazineacetamide;
N-4-Aminophenyl-N-4-methylbenzyl-4-methyl-1-piperazineacetamide;
N-4-Aminophenyl-N-2-hydroxybenzyl-4-methyl-1-piperazineacetamide;
N-4-Aminophenyl-N-4-methoxybenzyl-4-methyl-1-piperazineacetamide;
N-3-Aminophenyl-N-4-acetamidobenzyl-4-methyl-1-piperazineacetamide;
N-3-Aminophenyl-N-4-methoxybenzyl-4-methyl-1-piperazineacetamide;
N-3-Aminophenyl-N-4-methylbenzyl-4-methyl-1-piperazineacetamide;
N-4-Carboxyphenyl-N-4-methoxybenzyl-4-methyl-1-piperazineacetamide;
N-2-Hydroxymethylphenyl-N-benzyl-4-methyl-1-piperazineacetamide;
N-4-Ethoxycarbonylphenyl-N-2-hydroxybenzyl-4-methyl-1-piperazineacetamide;
N-4-Ethylphenyl-N-2-hydroxybenzyl-4-methyl-1-piperazineacetamide;
N-2-Carboxyphenyl-N-benzyl-4-methyl-1-piperazineacetamide;
N-4-Fluorophenyl-N-2-hydroxybenzyl-4-methyl-1-piperazineacetamide;
N-4-Chlorophenyl-N-2-hydroxybenzyl-4-methyl-1-piperazineacetamide;
N-4-Iodophenyl-N-2-hydroxybenzyl-4-methyl-1-piperazineacetamide;
N-4-Methoxyphenyl-N-2-hydroxybenzyl-4-methyl-1-piperazineacetamide;
N-2-Bromophenyl-N-2-hydroxybenzyl-4-methyl-1-piperazineacetamide;
N-3-Chlorophenyl-N-2-hydroxybenzyl-4-methyl-1-piperazineacetamide;
N-4-Bromophenyl-N-2-hydroxybenzyl-4-methyl-1-piperazineacetamide;
N-4-Iodo-phenyl-N-4-methylbenzyl-4-methyl-1-piperazineacetamide;
N-4-Dimethylaminophenyl-N-2-hydroxybenzyl-4-methyl-1-piperazineacetamide;
N-4-Chlorophenyl-N-4-methylbenzyl-4-methyl-1-piperazineacetamide;
N-4-Chlorophenyl-N-4-dimethylaminobenzyl-4-methyl-1-piperazineacetamide;
N-4-Chlorophenyl-N-4-methoxybenzyl-4-methyl-1-piperazineacetamide;
N-3-Trifluoromethylphenyl-N-benzyl-4-methyl-1-piperazineacetamide;
N-4-Fluorophenyl-N-4-acetamidobenzyl-4-methyl-1-piperazineacetamide;
N-4-Fluorophenyl-N-4-methoxybenzyl-4-methyl-1-piperazineacetamide;
N-4-Fluorophenyl-N-4-methyl-N-benzyl-4-methyl-1-piperazineacetamide;
N-4-Bromophenyl-N-4-methoxy-N-benzyl-4-methyl-1-piperazineacetamide;
N-4-Bromophenyl-N-4-methyl-N-benzyl-4-methyl-1-piperazineacetamide;
N-Phenyl-N-benzyl-4-methyl-1-piperazineacetamide;
N-(2-Pyridinyl)-N-4-methoxy-N-benzyl-4-methyl-1-piperazineacetamide;
N-(2-Pyridinyl)-N-benzyl-4-methyl-1-piperazineacetamide;
N-(3-Pyridinyl)-N-2-methoxybenzyl-4-methyl-1-piperazineacetamide; and
N-(3-Pyridinyl)-N-4-methylbenzyl-4-methyl-1-piperazineacetamide.

The compounds of the present invention are antimuscarinic agents, some of which have a selectivity for the M₁ receptor. "M₁ selectivity" means about a 5 fold greater potency at displacing selectively bound ligands from cloned M₁ receptors compared individually to other subtypes of cloned muscarinic receptors; preferably, there is a 50 fold difference and more preferably a 100 fold difference in the potency.

Some examples of compounds of the present invention which are believed to possess good M₁ selectivity are 4-amino-N,N-diphenyl-4-methyl-1-piperazineacetamide and N, N-diphenyl-4-methyl-1-piperazineacetamide.

Any appropriate test may be used in order to determine the affinity of the compounds of Formula I to muscarinic receptors or of specific subtypes of muscarinic receptors. For example to determine muscarinic subtype affinity, a pellet from cells A9L-m₁, A9L-m₂, A9L-m₃, A9L-m₄ or CHO-m₅ respectively for m₁, m₂, m₃, m₄, or m₅ subtypes, which are frozen at -80°C are thawed and homogenized in 10 ml of ice-cold 50 mM K/NaPO₄ buffer (pH 7.4) using a Polytron (setting 6 for 15 s). The homogenate is centrifuged at 18,000 rpm (40,000 g) in a Beckman centrifuge, rotor JA20 for 10 minutes at 4°C. The pellet is resuspended in the same buffer in order to have 0.5-2 mg protein/ml buffer. The incubation tubes contain 700 µl buffer, 100 µl atropine sulfate (2.10⁻⁶ M final) or test compound, 100 µl ³H-NMS ([³H]-N-methyl-scopolamine) (0.3 nM final) and 100 µl of homogenate. After a 1 hour incubation at room temperature, each incubation is terminated by rapid filtration through Whatman GF/B glass fiber filters, presoaked in water, using a Brandel cell harvester. The filters are rinsed 3 times with 5 ml 0.9% sodium chloride. The filters are transfered to scintillation vials and 10 ml of scintillation fluid (Quicksafe A) is added. The filters are counted by liquid scintillation spectrometry. Specific binding of ³H-NMS is measured as the excess over blanks taken in the presence of 2.10⁻⁶ M atropine sulfate. Protein content of the membranes is determined by the method of *J.Biol.Chem*. 193:265-275 (1951). Displacement curves are analyzed using the EBDA/LIGAND (Elsevier Biosoft) program to obtain Hill slopes and IC₅₀ values. The Kᵢ value is determined with the Cheng-Prusoff equation of *Biochem.Pharmacol.* 22:3099-3108 (1973) incorporated herein by reference.

The compounds of Formula I are useful in the treatment of Parkinson's disease. Parkinson's disease is a clinical syndrome comprised of bradykinesia, muscular rigidity, resting tremor and abnormalities of posture and gait. It afflicts approximately 1% of all adults over the age of 65 and usually is manifested after the age of 55. Without treatment, the patient succumbs to the disease typically due to complications of immobility such as pulmonary embolism, aspiration or hypostatic pneumonias. Its cause remains unknown, but it is generally responsive to anticholinergic and dopaminergic agents. It has been shown that many currently used anticholinergic antiparkinsonian drugs bind at various muscarinic receptors, for example see *Pharmacology & Toxicology* 62:90-94 (1988) incorporated herein by reference.

One method of demonstrating the use of the compounds of Formula I for the treatment of Parkinson's disease is found in the articles Pycock, C. J., *Neuroscience*, 5:461-514 (1980) and Ungerstedt, J., *Acta Physiol.Scand.* (Supp.) 367:69-93 (1971) incorporated herein by reference.

The treatment of a patient with Parkinson's disease with the compounds of Formula I is effected by administering to the patient an effective amount of a compound or combination of compounds of Formula I, preferably in combination with a pharmaceutically acceptable carrier, in order to treat the disease. This "effective amount" will vary with the extent of the disease state; the condition of the patient; variables between patients such as age, weight, concurrent medications taken, and concurrent disease states; and any other known factors typically considered when dosing medications. Typically, the compounds of Formula I used to treat Parkinsons's disease range from about 0.01 mg/kg to about 15 mg/kg.

The compounds of Formula I are useful in the treatment of motion sickness. Motion sickness is a disorder typically caused by repetitive angular, linear or vertical motion and is manifested in symptoms primarily of nausea and vomiting. Antimuscarinic agents have been tested in emesis models to show effectiveness in the control of vomiting, for example see *Neuropharmacology* 27(9):949-956 (1988). Antimuscarinics have also been shown useful in post-operative nausea and vomiting, and drug (such as opiate) induced nausea and vomiting. See Mitchelson, F., *Drugs* 43(3):295-315 (1992) incorporated herein by reference.

One method of demonstrating the use of the compounds of Formula I for the treatment of motion sickness is found in the article Lucot, J., *Pharm.Biochem.& Behavior*, 32:207-210 (1989) incorporated herein by reference.

The treatment of a patient with motion sickness with the compounds of the present invention is effected by administering to the patient an effective amount of a compound or combination of compounds of Formula I, preferably in combination with a pharmaceutically acceptable carrier, in order to treat the condition. This "effective amount" will vary with the extent of the disease state; the condition of the patient; variables between patients such as age, weight, concurrent medications taken, and concurrent disease states; and any other known factors typically considered when dosing medications. Typically, the compounds of Formula I used to treat motion sickness range from about 0.01 mg/kg to about 15 mg/kg. It is typical that the treatment of motion sickness also includes prophylactic treatment and it is preferably administered by transdermal administration.

The compounds of the present invention also inhibit the secretion of gastric acid. "Inhibition of gastric acid secretion" means to control gastric acidity by reducing the amount of gastric acid typically secreting by that individual. An example of a disease in which inhibition of gastric acid secretion is desirable is peptic ulcer disease. Patients with peptic ulcer disease generally have increased perietal cell mass, elevated basal and stimulated gastric acid and pepsin secretion, impaired suppression of gastrin release when the gastric contents are acidic, rapid gastric emptying, and/or a more acidic duodenal bulbar pH. Even though peptic ulcer disease is typically multifactorial, therapy directed towards reduction of gastric acid secretion has proven effective. Antimuscarinic agents, particularly M₁ antimuscarinic agents, have been shown to inhibit gastric acid secretion, for example see *European Journal of Pharmacology*, 112:211-224 (1985) incorporated herein by reference.

One method of demonstrating the use of the compounds of Formula I for the inhibition of gastric acid secretion is found in the article *European Journal of Pharmacology*, 112:211-224 (1985) incorporated herein by reference.

The treatment of a patient in need of an inhibition of gastric acid secretion with the compounds of the present invention is effected by administering to the patient an effective amount of a compound or combination of compounds of Formula I, preferably in combination with a pharmaceutically acceptable carrier, in order to treat the condition. This "effective amount" will vary with the extent of the disease state; the condition of the patient; variables between patients such as age, weight, concurrent medications taken, and concurrent disease states; and any other known factors typically considered when dosing medications. Typically, the amount of compounds of Formula I used to treat a patient in need of inhibition of gastric acid secretion is from about 0.01 mg/kg to about 15 mg/kg.

The duration of treatment using the compounds of formula I is highly variable and can be ascertained by those skilled in the art. The preferred route of administration is oral administration. For oral administration the formula I compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The Formula I compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as polyethylene glycol 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically acceptable adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl beta-aminopropionates, and 2-alkylimidazoline quarternary ammonium salts, as well as mixtures. The parenteral compositions of this invention will typically contain from about 0.5 to about 25% by weight of the formula I compound in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Patent Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Patent No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

For pharmacological end-use applications, the compounds of Formula I are preferentially administered in the form of their pharmaceutically acceptable acid addition salts. Compounds of Formula I can form pharmaceutically acceptable salts with any non-toxic organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salacylic, 2-phenoxybenzoic and sulfonic acids such as methane sulfonic, trifluoromethane sulfonic, 2-hydroxyethane sulfonic acid and p-toluenesulfonic acid.

## Claims

1. A compound of formula: 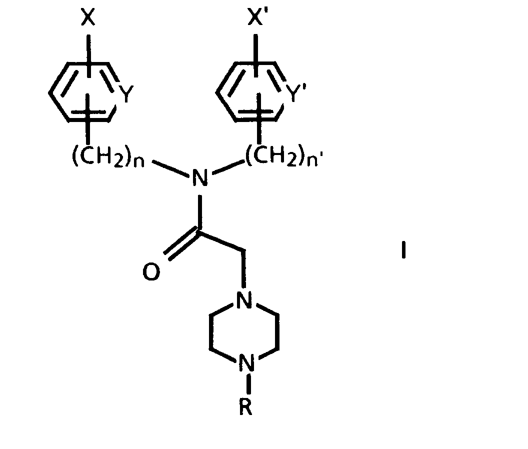 and the pharmaceutically acceptable salts thereof, wherein
each of X and X' are independently -H,-C₁-₄ alkyl, -Cl, -Br, -I, -F, -(CH₂)ₘCN, -(CH₂)ₘCOOR₁, -(CH₂)ₘCONR₂R₃, CF₃, -(CH₂)ₘNHQ, -(CH₂)ₘO(CH₂)_{p'}Z, -(CH₂)ₘNR₂R₃, -SO₃H, SO₂NR₂R₃, 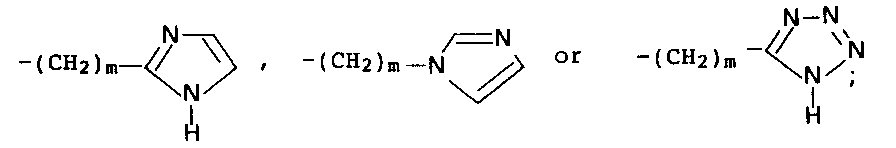 each of Y and Y' are independently -C- or -N-;
each of n, n' or m are independently 0 or 1;
p is 1, 2, 3, or 4;
p' is 0, 1, 2, 3, or 4;
R and R₁ are each independently H or C₁-C₄ alkyl;
each of R₂ and R₃ are independently H, -CH₃, -CH₂CH₃, C₄-C₇ cycloalkyl or taken together with the nitrogen atom form a heterocyclic ring having from 4 to 6 carbon atoms;
R₄ is C₁-C₄ alkyl;
Z is H, OH, -NR₂R₃, -C(O)OR₁, 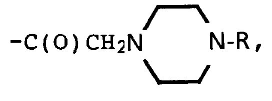 or 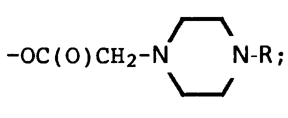 and
Q is -C(O)R₄, -(CH₂)_{p'}C(O)OR₁, -C(O)(CH₂)ₚC(O)OR₁, -C(O)CH=CHC(O)OR₁
or 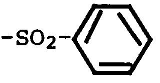 in which the phenyl moiety is optionally substituted with C₁-C₄ alkyl, C₁-C₄ alkoxy, CF₃ or NO₂,
provided that when Y or Y' are -N-, Y and Y' are unsubstituted,
provided that when Y is -N-, X is an ortho -NH₂ or H,
provided that when Y' is -N-, X' is an ortho -NH₂ or H,
provided that when Z is OH, -NR₂R₃ or 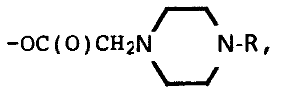 p' is 2, 3, or 4.

2. The compound of claim 1 wherein X is H and X' is NH₂.

3. The compound of claim 1 wherein X is H and X' is H.

4. The compound of claims 1, 2, or 3 wherein R is methyl.

5. The compound of claims 1, 2, 3, or 4 wherein n and n' are each zero.

6. The compound of claim 1 wherein the compound is N-4-Aminophenyl-N-phenyl-4-methyl-1-piperazineacetamide and pharmaceutically acceptable salts thereof.

7. The compound of claim 1 wherein the compound is N,N-diphenyl-4-methyl-1-piperazineacetamide and pharmaceutically acceptable salts thereof.

8. The compound according to claim 1 for use as a pharmaceutically active compound.

9. A pharmaceutical composition comprising the compound according to claim 1, optionally in combination with a pharmaceutically acceptable carrier.

10. The pharmaceutical composition according to claims 6 or 7 useful for the treatment of Parkinson's disease, motion sickness or for the inhibition of gastric acid secretion.

11. The compound according to claim 1 as pharmaceutically active agent for use in the treatment of Parkinson's disease, motion sickness or for the inhibition of gastric acid secretion.

12. Use of a compound according to claim 1, optionally in combination with a pharmaceutically acceptable carrier, for the preparation of a pharmaceutical composition for the treatment of Parkinson's disease, motion sickness or for the inhibition of gastric acid secretion.
